# EUROPEAN PATENT SPECIFICATION

(11) **EP 3 863 508 B1**
(45) Date of publication and mention of the grant of the patent: **02.12.2026**
(21) Application number: 19877904.3
(22) Date of filing: 31.10.2019
(51) Int. Cl.: A61B 5/00, A61B 5/024

(54) **APPARATUS AND METHOD FOR NON-INVASIVELY MEASURING PHYSIOLOGICAL PARAMETERS OF MAMMAL SUBJECT AND APPLICATIONS THEREOF**
VORRICHTUNG UND VERFAHREN ZUR NICHTINVASIVEN MESSUNG PHYSIOLOGISCHER PARAMETER VON SÄUGETIEREN UND ANWENDUNGEN DAVON
APPAREIL ET PROCÉDÉ DE MESURE NON INVASIVE DE PARAMÈTRES PHYSIOLOGIQUES D'UN SUJET MAMMIFÈRE ET LEURS APPLICATIONS

(30) Priority: 31.10.2018 US 201862753303 P; 31.10.2018 US 201862753453 P; 31.10.2018 US 201862753625 P; 04.06.2019 US 201962857179 P
(43) Date of publication of application: 18.08.2021
(73) Proprietor: Northwestern University, Evanston, IL 60208 (US)
(72) Inventor: ROGERS, John, A., Wilmette, IL 60091 (US); CHUNG, Ha, Uk, Evanston, IL 60208 (US); RWEI, Alina, Evanston, IL 60208 (US); HOURLIER-FARGETTE, Aurelie, Evanston, IL 60208 (US); LIU, Claire, Evanston, IL 60208 (US); LEE, Kun, Hyuck, Evanston, IL 60208 (US); CARLINI, Andrea, S., Evanston, IL 60208 (US); XU, Shuai, Bala Cynwyd, PA 19004 (US); RYU, Dennis, Evanston, IL 60208 (US); LEE, Jong, Yoon, Evanston, IL 60208 (US)
(74) Representative: Weickmann & Weickmann PartmbB
(86) International application number: PCT/US2019/059190
(87) International publication number: WO 2020/092786

(56) References cited:
- WO-A1-2018/013569
- WO-A1-2018/136462
- KR-A- 20160 007 056
- US-A1- 2017 071 550
- US-A1- 2017 325 724
- US-A1- 2017 347 894
- US-A1- 2017 347 894
- US-A1- 2018 014 734

## Description

### CROSS-REFERENCE TO RELATED APPLICATIONS

This PCT application claims priority to and the benefit of U.S. Provisional Patent Application Serial Nos. 62/753,303, 62/753,453 and 62/753,625, each of which was filed October 31, 2018, and U.S. Provisional Patent Application Serial No. 62/857,179, which was filed June 4, 2019.

This PCT application is related to a co-pending PCT patent application entitled "APPARATUS AND METHOD FOR MEASURING PHYSIOLOGICAL PARAMETERS OF MAMMAL SUBJECT AND APPLICATIONS OF SAME", by John A. Rogers et al., with Attorney Docket No. 0116936.213WO2, a co-pending PCT patent application entitled "SENSOR NETWORK FOR MEASURING PHYSIOLOGICAL PARAMETERS OF MAMMAL SUBJECT AND APPLICATIONS OF SAME", by John A. Rogers et al., with Attorney Docket No. 0116936.214WO2, and a co-pending U.S. patent application entitled "APPARATUS AND METHOD FOR NON-INVASIVELY MEASURING BLOOD PRESSURE OF MAMMAL SUBJECT", by John A. Rogers et al., with Attorney Docket No. 0116936.215US2, each of which is filed on the same day that this PCT application is filed, and with the same assignee as that of this application.

Some references, which may include patents, patent applications and various publications, are cited and discussed in the description of this invention. The citation and/or discussion of such references is provided merely to clarify the description of the present invention and is not an admission that any such reference is "prior art" to the invention described herein.

### FIELD OF THE INVENTION

The present invention relates generally to healthcare, and more particularly to apparatuses and methods for non-invasively measuring physiological parameters of a mammal subject and applications of the same.

### BACKGROUND OF THE INVENTION

The background description provided herein is for the purpose of generally presenting the context of the invention. The subject matter discussed in the background of the invention section should not be assumed to be prior art merely as a result of its mention in the background of the invention section. Similarly, a problem mentioned in the background of the invention section or associated with the subject matter of the background of the invention section should not be assumed to have been previously recognized in the prior art. The subject matter in the background of the invention section merely represents different approaches, which in and of themselves may also be inventions. Work of the presently named inventors, to the extent it is described in the background of the invention section, as well as aspects of the description that may not otherwise qualify as prior art at the time of filing, are neither expressly nor impliedly admitted as prior art against the invention.

Current neonatal and pediatric critical care is complicated by involving multiple wired devices, often with an invasive catheter, for measuring health condition continuously. For example, in the United States, over 480,000 critically-ill infants and children are admitted to intensive care units each year, with infants less than one year of age suffering from the highest mortality rate among age groups below 19 years old and requiring more intensive care compared to older children. Furthermore, every year 300,000 neonates are admitted to the NICU in the U.S, with the market is expected to reach $11.86 billion by 2022. These fragile patients include premature infants that may weigh as little as 500g (1.1 lbs), while the term baby would weigh about seven times more. Continuous monitoring of vital signs is essential for critical care, yet existing technologies require the use of multiple leads and skin-contacting interfaces with hard-wires connected to electronic processing systems that are often tethered to the wall, obstructing the effectiveness of clinical care, making it difficult to perform therapeutic skin-to-skin contact, called kangaroo mother care (KMC), thus impeding psychological bonding between the parent and child. Thus, continuous monitoring of vital signs in the neonatal and pediatric intensive care units generally requires multiple wired devices applied onto the skin and invasive techniques such as arterial line, elevating the risk of complications and impeding the opportunity for skin-to-skin therapy. Thus, new technology is required to meet the unique demands.

Therefore, a heretofore unaddressed need exists in the art to address the aforementioned deficiencies and inadequacies. Relevant prior art is disclosed in WO 2018/013569 A1, WO 2018/136462 A1 and US 2017/347894 A1.

### SUMMARY OF THE INVENTION

According to a first aspect of the present invention there is provided an apparatus for non-invasively measuring physiological parameters of a mammal subject, comprising:
a plurality of sensor systems attached to the neonatal subject, wherein the plurality of sensor systems are time-synchronized and communicate with each other wirelessly and bidirectionally, wherein the plurality of sensor systems comprises:
a first epidermal electronic system (EES) attached to a torso region of the neonatal subject, the first EES comprising an electrocardiography (ECG) sensor, an accelerometer sensor, a temperature sensor, a plurality of flexible and stretchable interconnects comprising serpentine or zig-zag interconnects, and an elastomeric encapsulation layer;
a second EES attached to a limb region of the neonatal subject, the second EES comprising a photoplethysmography (PPG) sensor, a temperature sensor, a plurality of flexible and stretchable interconnects comprising serpentine or zig-zag interconnects, and an elastomeric encapsulation;
wherein the first and second EES are configured to detect physiological signals that are time-synchronized with each other and to wirelessly communicate with each other; and
a microcontroller unit (MCU) adapted in wireless communication with the plurality of sensor systems, and configured to wirelessly receive the time-synchronized physiological signals from the first and second EES and to determine one or more physiological parameters of the neonatal subject based on the physiological signals, from the sensor systems, and to display the physiological parameters of the mammal subject.

According to a second aspect of the present invention there is provided a method of non-invasively measuring physiological parameters of a neonatal subject, the method comprising:
attaching a first EES epidermal electronic system (EES) comprising an electrocardiography (ECG) sensor, an accelerometer sensor, a temperature sensor, flexible and stretchable serpentine or zig-sag interconnects, and an elastomeric encapsulation layer to a torso region of the neonatal subject;
attaching a second EES comprising a photoplethysmography (PPC) sensor, a temperature sensor, flexible and stretchable serpentine or zig-zag interconnects, and an elastomeric encapsulation layer to a limb region of the neonatal subject;
detecting physiological signals at each of the first and second EES, the physiological signals being time-synchronized across the first and second EES;
wirelessly transmitting the physiological signals from the EES to a microcontroller unit; and determining, at the microcontroller, one or more physiological parameters of the mammal subject based on the physiological signals.

### SUMMARY OF THE DISCLOSURE

One of the objectives of the disclosure is to provide an apparatus for non-invasively measuring physiological parameters of a mammal subject, which may be used as a vital sign monitoring system and/or a pediatric medical device, a method thereof, and applications thereof.

In one aspect, the disclosure relates to an apparatus for non-invasively measuring physiological parameters of a mammal subject. In certain embodiments, the apparatus includes: a plurality of sensor systems attached to the mammal subject, wherein the sensor systems are time-synchronized and communicate with each other wirelessly and bidirectionally, wherein each of the sensor systems comprises at least one sensor configured to detect a vital sign of the mammal subject and generate a corresponding one of the physiological parameters; and a microcontroller unit (MCU) adapted in wireless communication with the plurality of sensor systems, and configured to receive, from the sensor systems, and to display the physiological parameters of the mammal subject.

In one embodiment, the sensor is configured to detect the vital sign as a signal including one of: an electrical signal related to at least one of electrocardiography (ECG) and electromyography (EMG) technology; a mechanical signal related to movement, respiration and arterial tonometry; an acoustic signal related to vocal cord vocalization, respiratory sound and heart sound; and an optical signal related to blood oxygenation.

In one embodiment, each of the sensor systems is an epidermal electronic system (EES) comprising: a plurality of electronic components, and a plurality of flexible and stretchable interconnects electrically connected to different electronic components; and an elastomeric encapsulation layer at least partially surrounding the electronic components and the flexible and stretchable interconnects to form a tissue-facing surface attached to the mammal subject and an environment-facing surface. In one embodiment, the plurality of flexible and stretchable interconnects comprise at least one of serpentine interconnects and zigzag interconnects. In one embodiment, each of the sensor systems further comprises a foldable electronic board, wherein the plurality of electronic components and the plurality of flexible and stretchable interconnects are disposed on the foldable electronic board.

In one embodiment, the sensor systems comprise: a first EES disposed in a torso region of the mammal subject; and a second EES disposed in a limb region of the mammal subject. In one embodiment, the first EES is an electrocardiography (ECG) EES, and the electronic components of the ECG EES comprise at least two electrodes spatially apart from each other for ECG generation. In one embodiment, the second EES is a photoplethysmography (PPG) EES, and the electronic components of the PPG EES comprise a PPG sensor comprising an optical source and an optical detector located within a sensor footprint. In one embodiment, the electronic components of each of the sensor systems comprise a thermometer.

In one embodiment, each of the sensor systems further comprises a power supply, and the power supply is an embedded power supply or a detachable modular power supply.

In one embodiment, the sensor systems comprise: a first sensor system disposed in a torso region of the mammal subject, wherein the first sensor system is an inertial motion sensor system or an accelerometer system; and a second sensor system disposed in a limb region of the mammal subject, wherein the second sensor system is a photoplethysmography (PPG) epidermal electronic system (EES).

In one embodiment, each of the sensor systems is in wireless communication with the MCU via a near field communication (NFC) protocol, or Bluetooth protocol. In one embodiment, each of the sensor systems comprises a magnetic coil in compliance with the NFC protocol to allow wireless data transmission and wireless power transmission through a single link.

In one embodiment, each of the sensor systems further comprises one or more of: an accelerometer for position or movement monitoring; and a temperature sensor for measuring temperature.

In one embodiment, each of the sensor systems is waterproof.

In one embodiment, the physiological parameters of the mammal subject comprise one or more of: heart rate, heart rate variability, heart sounds, blood pressure, chest wall displacement, electromyography, electrocardiography, blood oxygenation, respiratory rate, respiratory effort, respiratory cadence, tidal volume, coughing, snoring, sneezing, throat clearing, wheezing, apnea, hypoapnea, physical activity, core body position, peripheral limb position, scratching, vocalizations, rubbing, walking, sleep quality, sleep time, wake time upon sleeping, skin temperature, core body temperature, and a combination thereof.

In one embodiment, the blood pressure is measured by: receiving output signals of a first sensor disposed in a first position of the mammal subject and a second sensor disposed in a second position of the mammal subject; processing the output signals to determine a pulse arrival time (PAT) as a time delay Δt between detection of a first signal by the first sensor and detection of a second signal by the second sensor; determining a pulse wave velocity (PWV) based on the PAT and a pulse arrival distance L between the first position and the second position, wherein $PWV = \frac{L}{Δ t}$ ; and determining the blood pressure P of the mammal subject from the PWV, wherein P = αPWV² + β, and α and β are empirically determined constants depending on artery geometry and artery material properties of the mammal subject. In one embodiment, at a blood pressure range between 5 kPA and 20 kPa, $0.13 kPa \times {s}^{2} / {m}^{2} \leq α \leq 0.23 kPa \times {s}^{2} / {m}^{2} ;$ and $2.2 kPa \leq β \leq 3.2 kPa .$

In one embodiment, the mammal subject is a human subject or a non-human subject.

In another aspect, the disclosure relates to a method for developing vaccines for a disease on a mammal subject, including: providing a vaccine agent to the mammal subject not having the disease; monitoring, continuously for a period of time, physiological parameters of the mammal subject using the apparatus as discussed above; and evaluating effects of the vaccine agent on the mammal subject in the period of time based on the physiological parameters.

In yet another aspect, the disclosure relates to a method for developing therapeutics for a disease on a mammal subject, including: providing a therapeutic agent to the mammal subject having the disease; monitoring, continuously for a period of time, physiological parameters of the mammal subject using the apparatus as discussed above; and evaluating effects of the therapeutic agent on the disease in the period of time based on the physiological parameters.

In a further aspect, the disclosure relates to a method for diagnosing a disease on a mammal subject, including: monitoring, continuously for a period of time, physiological parameters of the mammal subject using the apparatus as discussed above; and determining whether the mammal subject has the disease based on the physiological parameters.

In one embodiment, the method further includes performing a corresponding treatment of the disease based on the physiological parameters. In one embodiment, the treatment includes providing a respiratory medicine to the mammal subject.

In yet a further aspect, the disclosure relates to a method of non-invasively measuring physiological parameters of a mammal subject, including: utilizing a plurality of sensor systems on the mammal subject, wherein the sensor systems are time-synchronized and communicate with each other wirelessly and bidirectionally, and each of the sensor systems comprises at least one sensor to monitor one of the physiological parameters; measuring, by the sensor systems, the physiological parameters of the mammal subject; receiving, at a microcontroller remotely communicatively connected to the sensor systems, the physiological parameters of the mammal subject; and displaying, at the microcontroller, the physiological parameters of the mammal subject.

In one embodiment, the sensor is configured to detect a vital sign of the mammal subject as a signal selected from a group consisting of: an electrical signal related to at least one of electrocardiography (ECG) and electromyography (EMG) technology; a mechanical signal related to movement, respiration and arterial tonometry; an acoustic signal related to vocal cord vocalization, respiratory sound and heart sound; and an optical signal related to blood oxygenation.

In one embodiment, each of the plurality of sensor systems is an epidermal electronic system (EES) comprising: a plurality of electronic components, and a plurality of flexible and stretchable interconnects electrically connected to different electronic components; and an elastomeric encapsulation layer at least partially surrounding the electronic components and the flexible and stretchable interconnects to form a tissue-facing surface attached to the mammal subject and an environment-facing surface. In one embodiment, the plurality of flexible and stretchable interconnects comprise at least one of serpentine interconnects and zigzag interconnects. In one embodiment, each of the sensor systems further comprises a foldable electronic board, wherein the plurality of electronic components and the plurality of flexible and stretchable interconnects are disposed on the foldable electronic board.

In one embodiment, the plurality of sensor systems comprise: a first EES disposed in a torso region of the mammal subject; and a second EES disposed in a limb region of the mammal subject. In one embodiment, the first EES is an electrocardiography (ECG) EES and comprises at least two electrodes spatially apart from each other for ECG generation. In one embodiment, the second EES is a photoplethysmography (PPG) EES and comprises a PPG sensor comprising an optical source and an optical detector located within a sensor footprint.

In one embodiment, the sensor systems comprise: a first sensor system disposed in a torso region of the mammal subject, wherein the first sensor system is an inertial motion sensor system or an accelerometer system; and a second sensor system disposed in a limb region of the mammal subject, wherein the second sensor system is a photoplethysmography (PPG) epidermal electronic system (EES).

In one embodiment, the physiological parameters of the mammal subject comprise one or more of: heart rate, heart rate variability, heart sounds, blood pressure, chest wall displacement, electromyography, electrocardiography, blood oxygenation, respiratory rate, respiratory effort, respiratory cadence, tidal volume, coughing, snoring, sneezing, throat clearing, wheezing, apnea, hypoapnea, physical activity, core body position, peripheral limb position, scratching, vocalizations, rubbing, walking, sleep quality, sleep time, wake time upon sleeping, skin temperature, core body temperature, and a combination thereof.

In one embodiment, the blood pressure is measured by: receiving output signals of a first sensor disposed in a first position of the mammal subject and a second sensor disposed in a second position of the mammal subject; processing the output signals to determine a pulse arrival time (PAT) as a time delay Δt between detection of a first signal by the first sensor and detection of a second signal by the second sensor; determining a pulse wave velocity (PWV) based on the PAT and a pulse arrival distance L between the first position and the second position, wherein $PWV = \frac{L}{Δ t}$ ; and determining the blood pressure P of the mammal subject from the PWV, wherein P = αPWV² + β, and α and β are empirically determined constants depending on artery geometry and artery material properties of the mammal subject. In one embodiment, at a blood pressure range between 5 kPA and 20 kPa, $0.13 kPa \times {s}^{2} / {m}^{2} \leq α \leq 0.23 kPa \times {s}^{2} / {m}^{2} ;$ and $2.2 kPa \leq β \leq 3.2 kPa .$

In one embodiment, each of the plurality of sensor systems further comprises a power supply, and the power supply is an embedded power supply or a detachable modular power supply.

In one embodiment, each of the plurality of sensor systems is in wireless communication with the microcontroller via a near field communication (NFC) protocol, or Bluetooth protocol.

In one embodiment, each of the plurality of sensor systems further comprises one or more of: an accelerometer for position or movement monitoring; and a temperature sensor for measuring temperature. In one embodiment, each of the plurality of sensor systems comprises a magnetic coil in compliance with the NFC protocol to allow wireless data transmission and wireless power transmission through a single link.

In a further aspect, the disclosure relates to a non-transitory tangible computer-readable medium storing instructions which, when executed by one or more processors, cause the method as discussed above to be performed.

These and other aspects of the disclosure will become apparent from the following description of the preferred embodiment taken in conjunction with the following drawings, although variations and modifications therein may be affected without departing from the scope of the novel concepts of the invention.

### BRIEF DESCRIPTION OF THE DRAWINGS

The following drawings form part of the present specification and are included to further demonstrate certain aspects of the invention. The invention may be better understood by reference to one or more of these drawings in combination with the detailed description of specific embodiments presented herein. The drawings described below are for illustration purposes only. The drawings are not intended to limit the scope of the present teachings in any way.
FIG. 1 schematically shows a functional block diagram of an apparatus according to certain embodiments of the present disclosure.
FIGS. 2A-2D show schematic illustrations and photographic images of ultra-thin, skin-like wireless modules in the apparatus for measuring the physiological parameters in the neonatal intensive care unit (NICU) with comparisons to clinical standard instrumentation, according to embodiments of the disclosure.

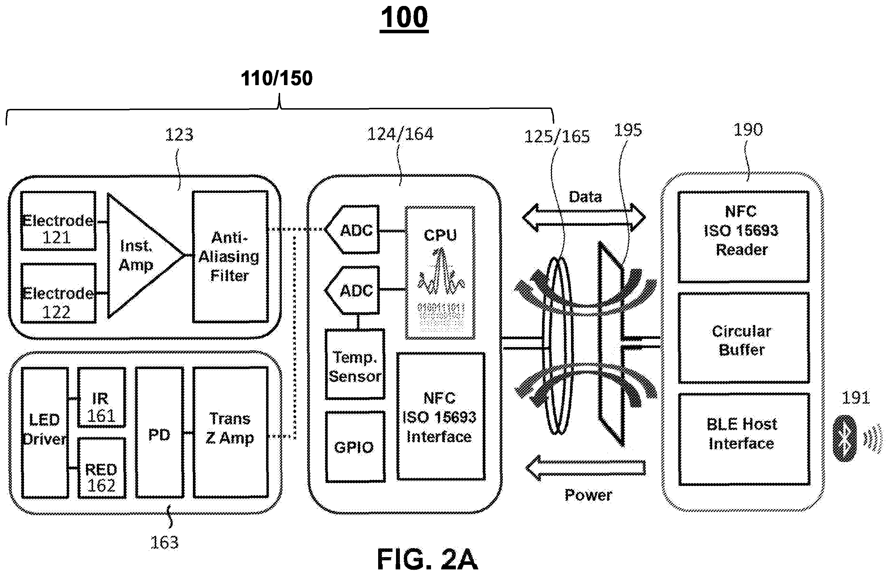

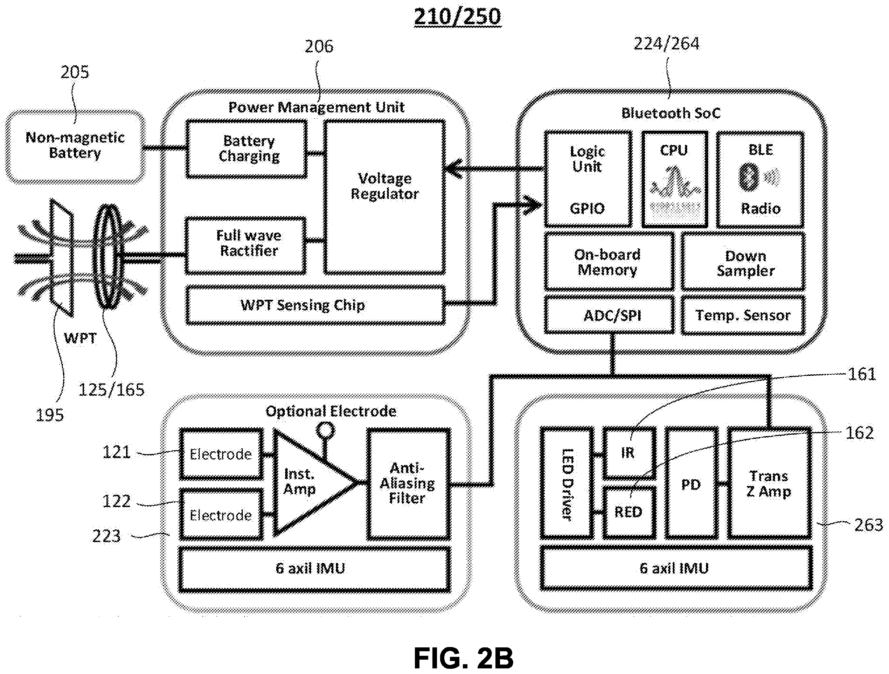

FIG. 2A is a functional block diagram showing analog front end and electronic components of each EES, components of the near-field communication (NFC) system on a chip (SoC) including microcontroller, general-purpose input/output (GPIO), and radio interface, with a host reader platform that includes an NFC reader module and a Bluetooth low energy (BLE) interface with circular buffer. FIG. 2B shows a functional block diagram of the two sensor systems according to another embodiment of the disclosure.

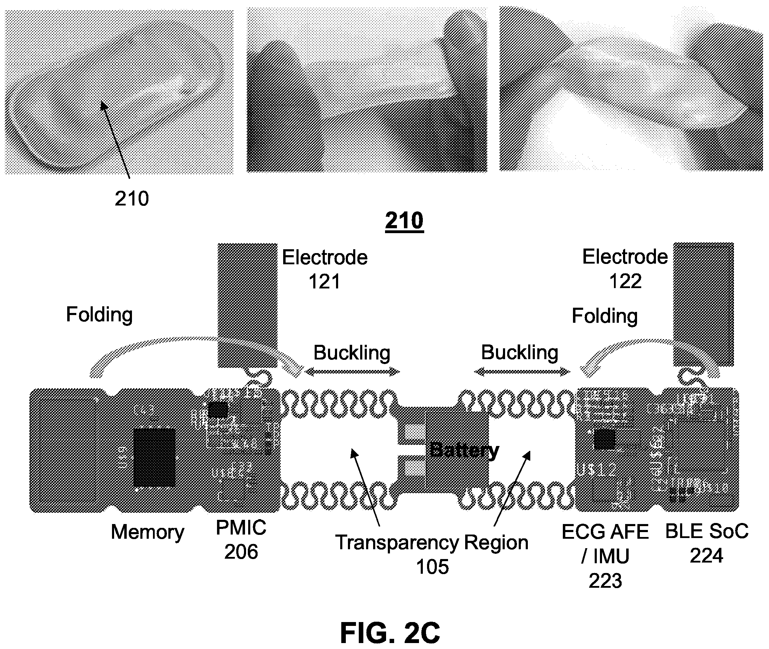

FIG. 2C is a schematic of a sensor system configured to mount on the torso, such as a chest, according to one embodiment of the disclosure.

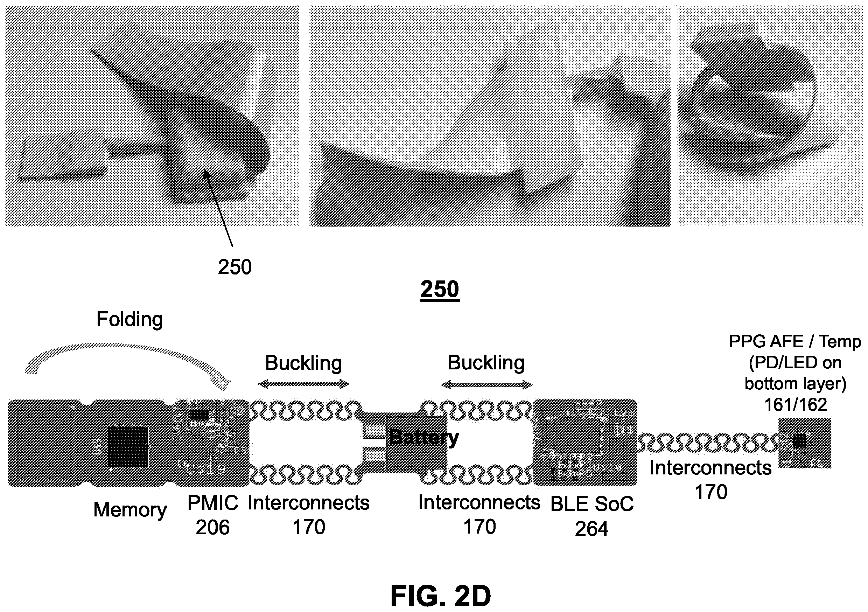

FIG. 2D shows a sensor system configured to mount on an extremity, such as a foot, leg, hand, arm finger, toe or nail, such as by a wrapping-type mechanism to secure the main circuit components with a mechanically decoupled sensor system connected thereto, according to one embodiment of the disclosure.

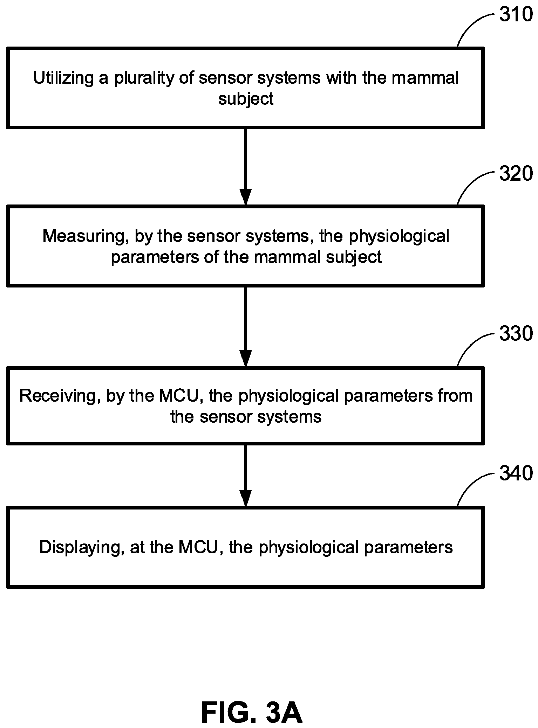

FIG. 3A shows a flowchart of a method of non-invasively and continuously measuring physiological parameters of a mammal subject according to certain embodiments of the present disclosure.

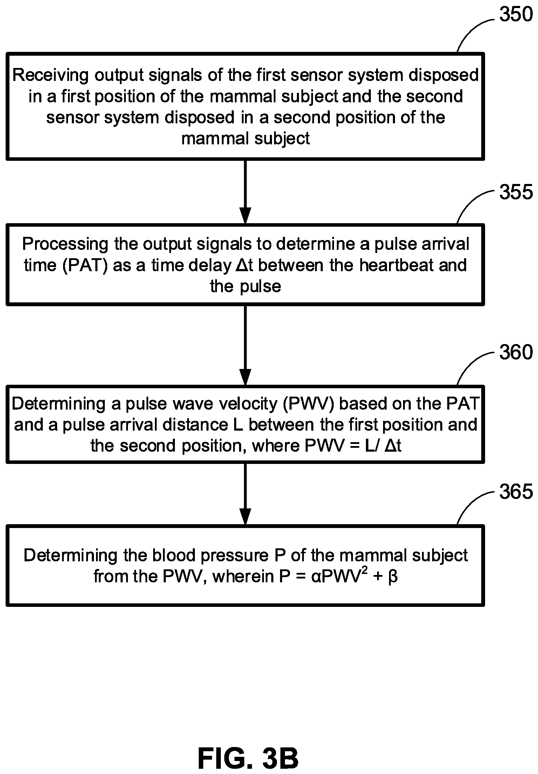

FIG. 3B shows a flowchart of a method of non-invasively and continuously measuring blood pressure of a mammal subject according to certain embodiments of the present invention.

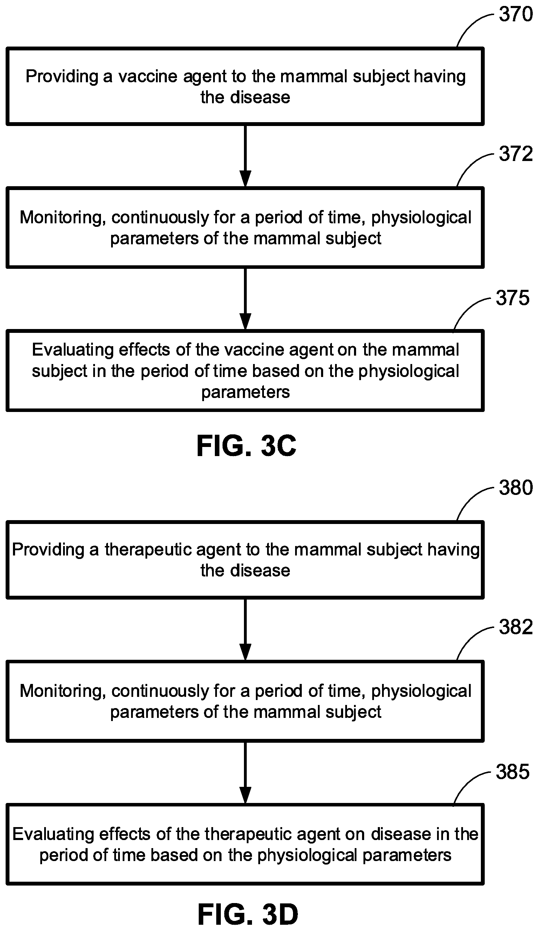

FIG. 3C shows a flowchart of a method developing vaccines for a disease on a mammal subject according to certain embodiments of the present disclosure.
FIG. 3D shows a flowchart of a method for developing therapeutics for a disease on a mammal subject according to certain embodiments of the present disclosure.

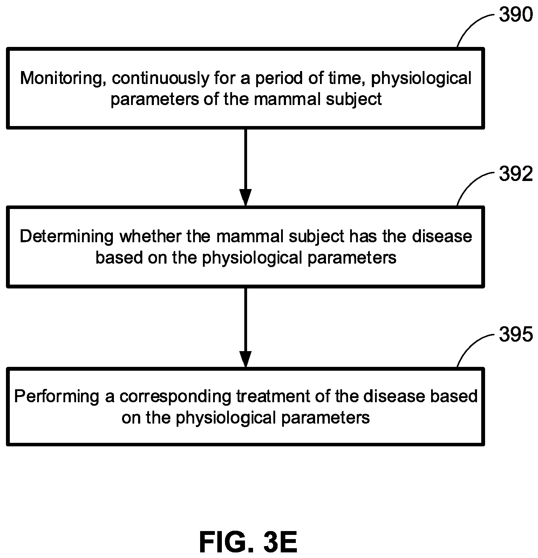

FIG. 3E shows a flowchart of a method for diagnosing a disease on a mammal subject according to certain embodiments of the present disclosure.

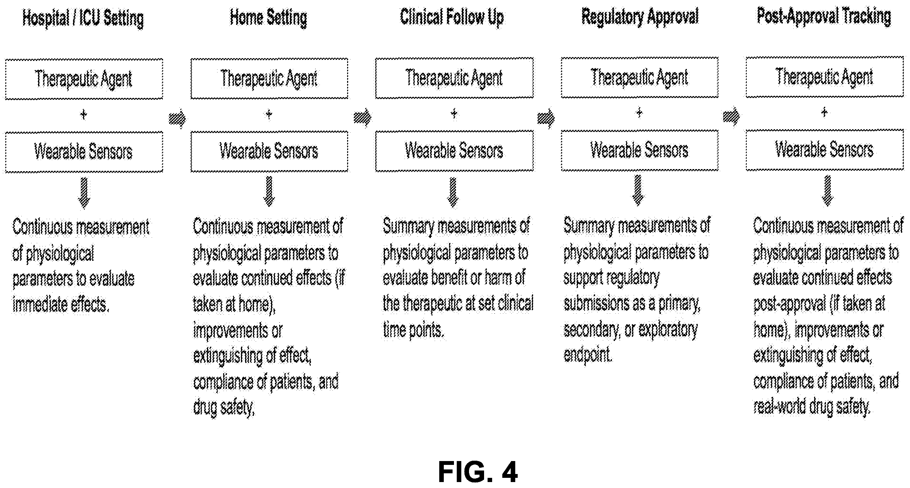

FIG. 4 shows a flow diagram illustrating use of wearable sensor technology to support supports the development, testing, approval, and post-market tracking of a wide range of therapeutic agents according to certain embodiments of the present disclosure.

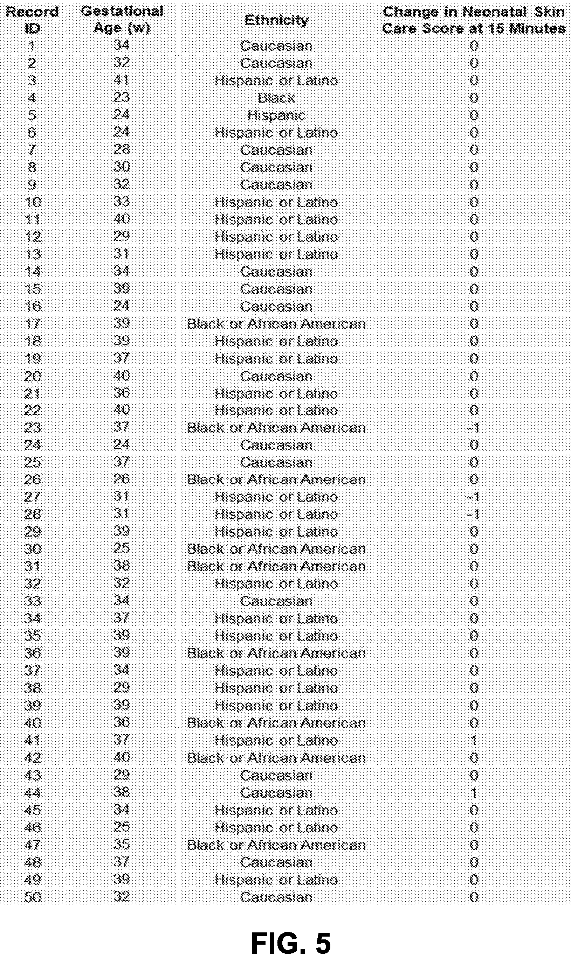

FIG. 5 shows a table of clinical characteristics of neonates admitted in the NICU/PICU according to certain embodiments of disclosure.

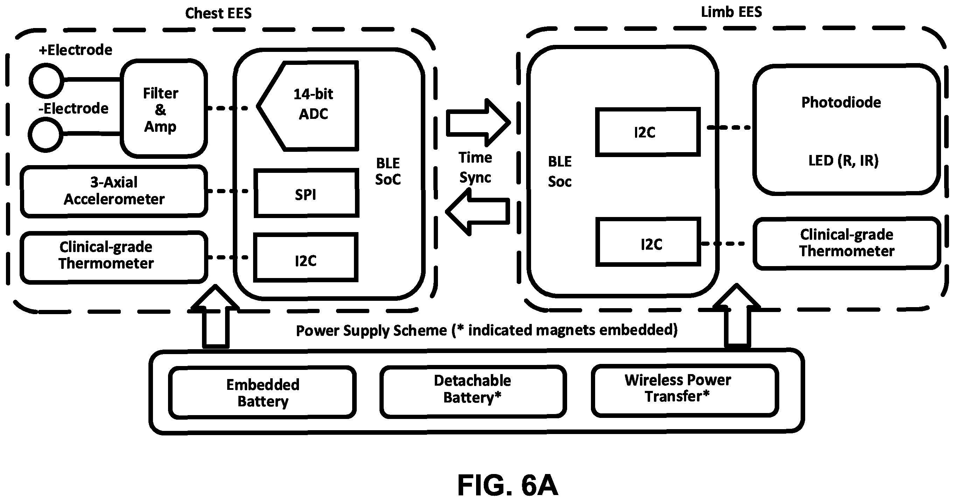

FIG. 6A schematically shows a functional block diagram of core components of an apparatus in accordance with the present invention including two time-synchronized EES including analog-front-end for ECG processing, 3-axis accelerometer, thermometer IC, and the BLE SoC for the Chest EES and pulse oximeter IC, thermometer, and the BLE SoC for the Limb EES.

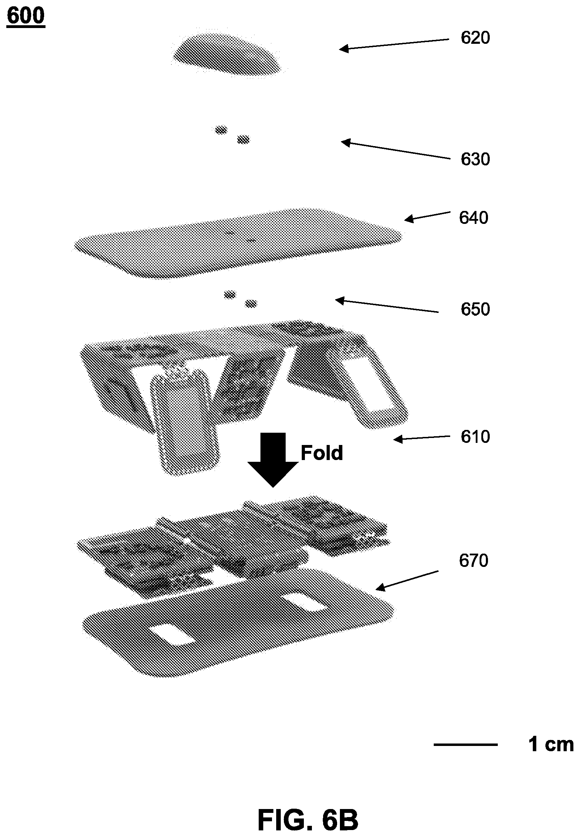

FIG. 6B schematically shows an exploded view of a chest EES sensor with the embedded battery modular power supply options according to certain embodiments of disclosure.

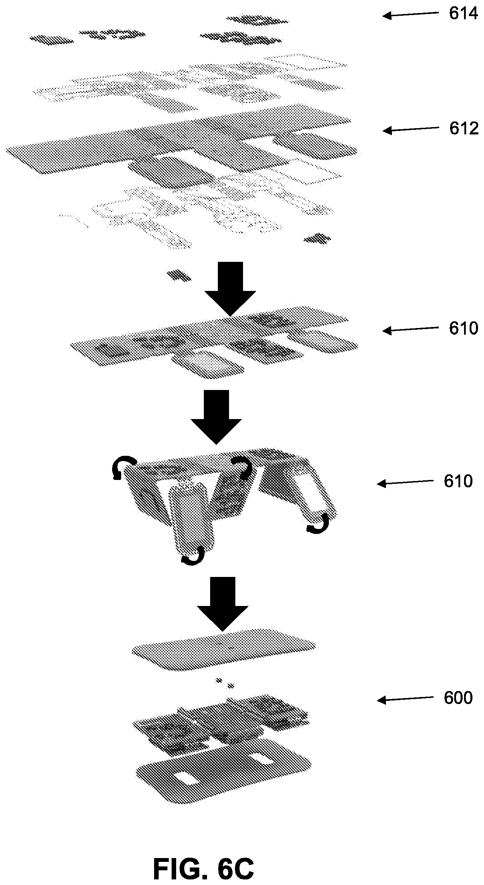

FIG. 6C schematically shows the formation of the chest EES sensors as shown in FIG. 6B.

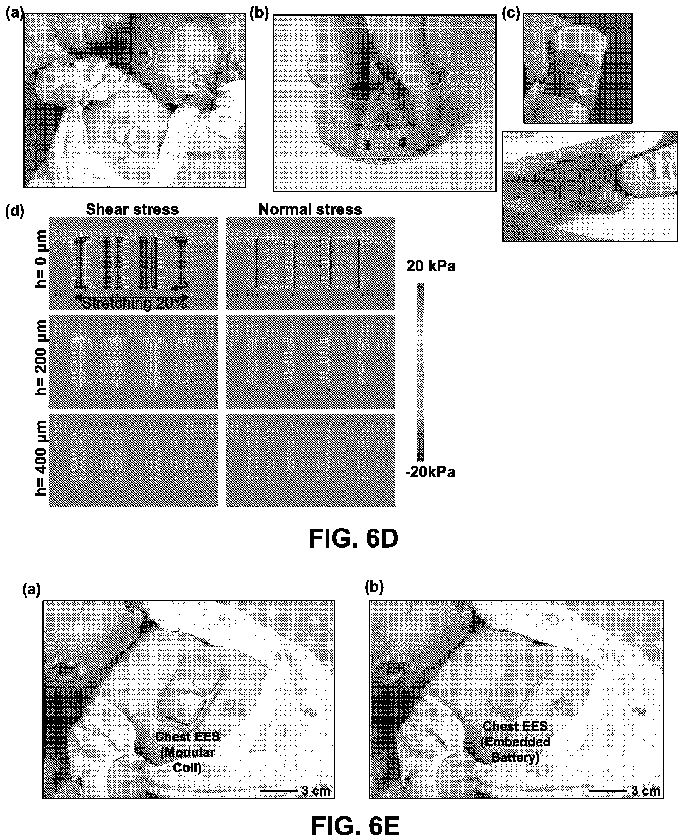

FIG. 6D schematically shows examples of the flexible and wireless sensors according to certain embodiments of the disclosure, where panel (a) shows a photographic image of the Chest EES on a realistic baby doll, panel (b) shows the waterproof feature of the EES, panel (c) shows photographic images of the overall Limb EES FPCB bent around wrist-to-base of the foot interface, and panel (d) shows mechanics of the Chest EES FPCB when the interconnects are stretched.
FIG. 6E shows photographic image of the Chest EES on a realistic baby doll with panel (a) a modular coil Chest EES version and panel (b) an embedded battery version according to certain embodiments of the disclosure.

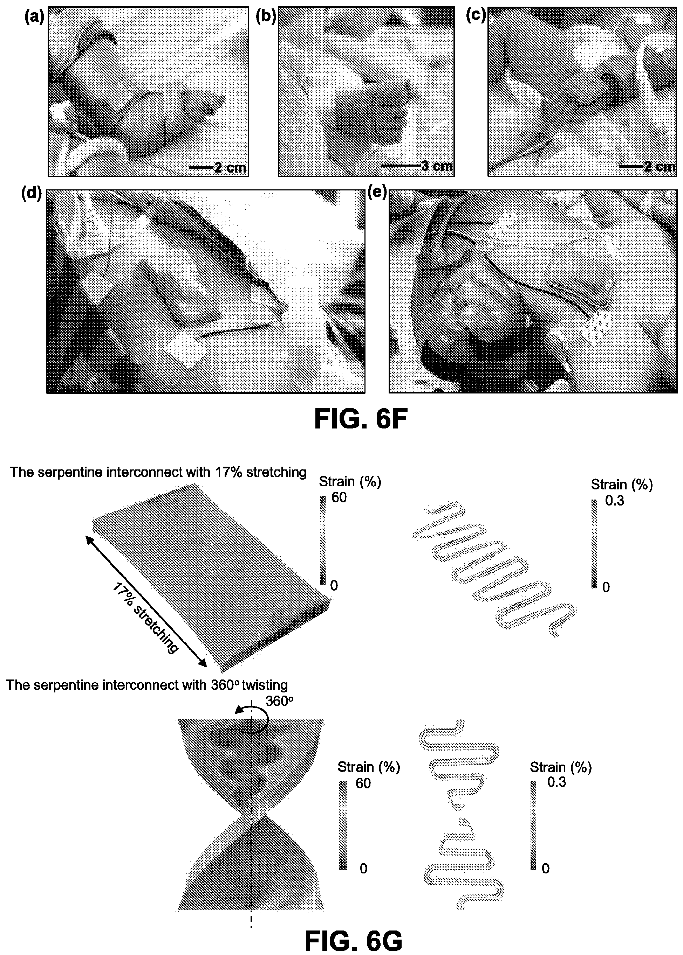

FIG. 6F shows photographic image of deployment of the Chest EES and the Limb EES according to certain embodiments of the disclosure, where panel (a) shows deployment of the Limb EES on a NICU baby at the wrist-to-base of the foot interface, panel (b) shows deployment of the Limb EES on a PICU baby at the foot-to-toe interface, panel (c) shows deployment of the Limb EES on a PICU baby at the wrist-to-hand interface, panel (d) shows deployment of the Chest EES on a PICU baby having a respiration disease with a defeated chest, and panel (e) shows deployment of the Chest EES on a NICU baby with a defeated chest.
FIG. 6G shows the stretching and bending characteristics of the serpentine interconnects of the Limb EES that is optimized up to the bending radius of 3.9 mm according to certain embodiments of the disclosure.

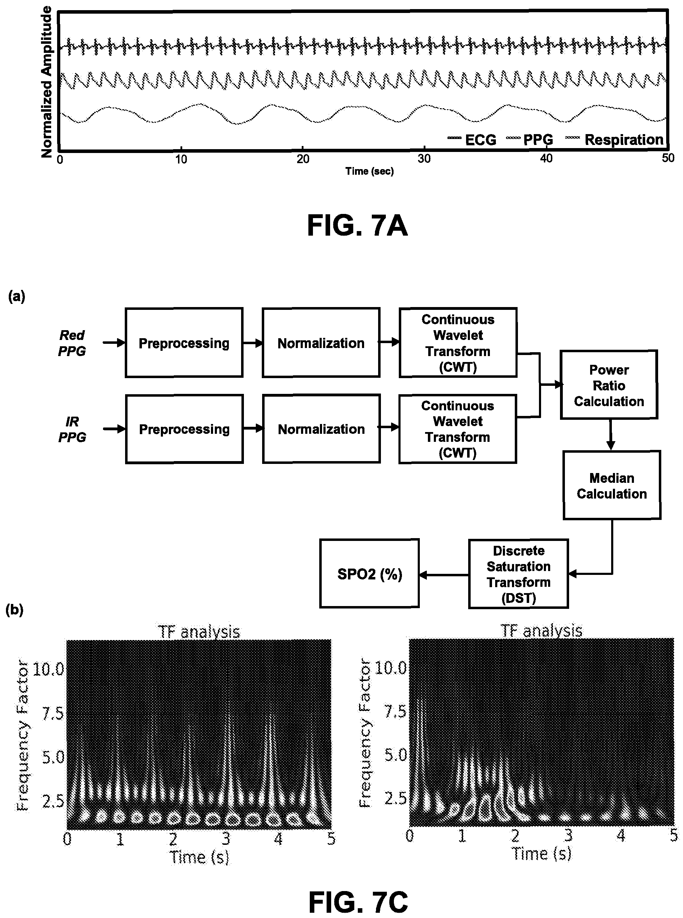

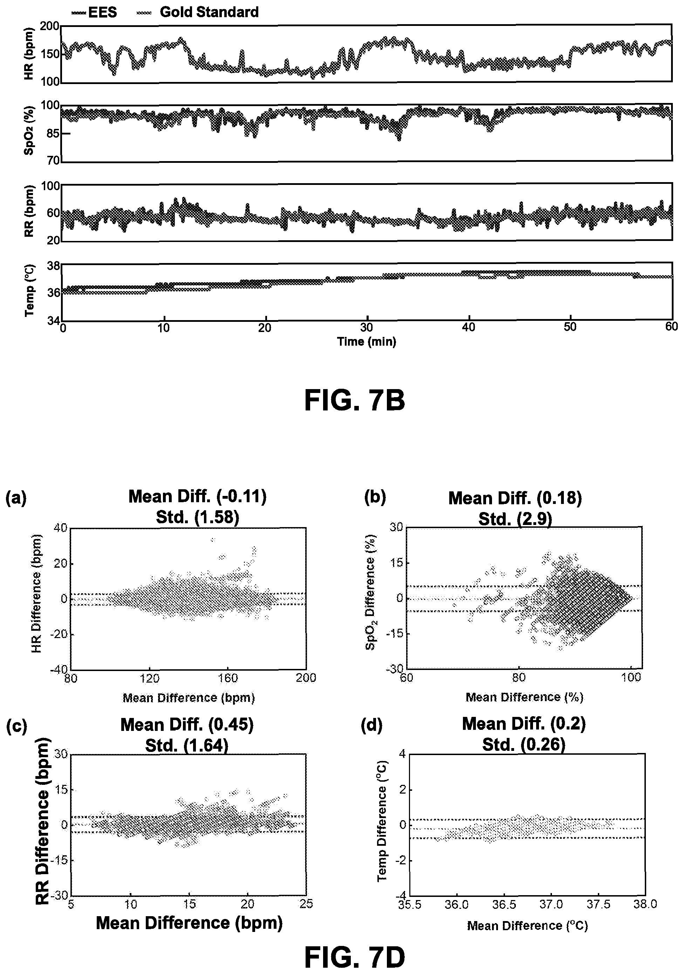

FIGS. 7A-7D shows data collection in the neonatal/pediatric intensive care units according to certain embodiments of the disclosure. FIG. 7A shows Representative ECG, PPG and respiration waveforms collected by EES real-time from a neonate (GA: wks). FIG. 7B shows representative comparison of vital signs captured by EES including HR, SpO₂, RR, and temperature to clinical gold standard. FIG. 7C shows panel (a) signal processing algorithms in SpO₂ and panel (b) two different results of the signal processing. FIG. 7D shows representative figures for safety related to heat generation of the device during a 24-hour operation, where panel (a) shows a chest unit did not create any significant heating after 24-hr operation, and panel (b) shows a limb unit did not create any significant heating after 24-hr operation.

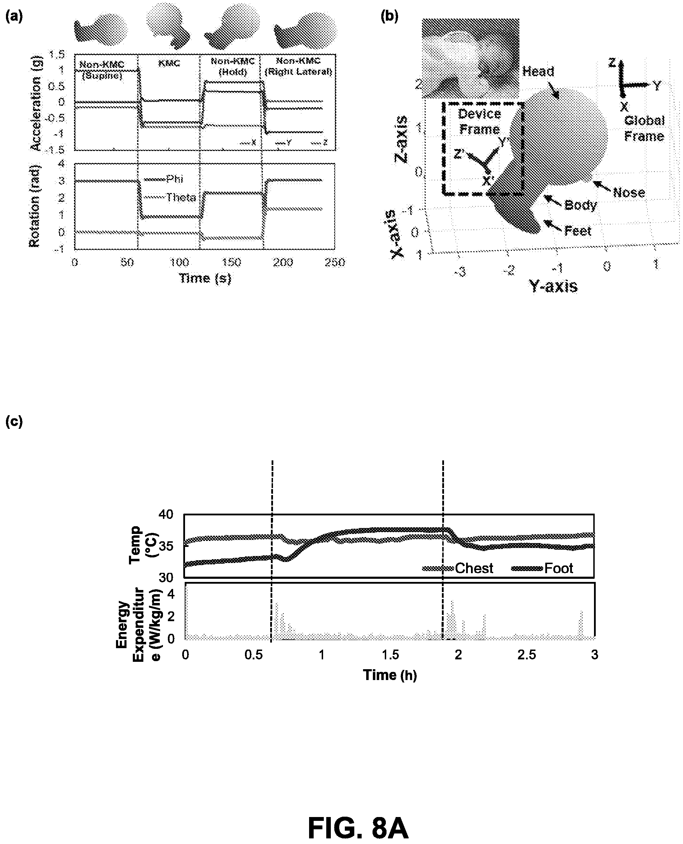

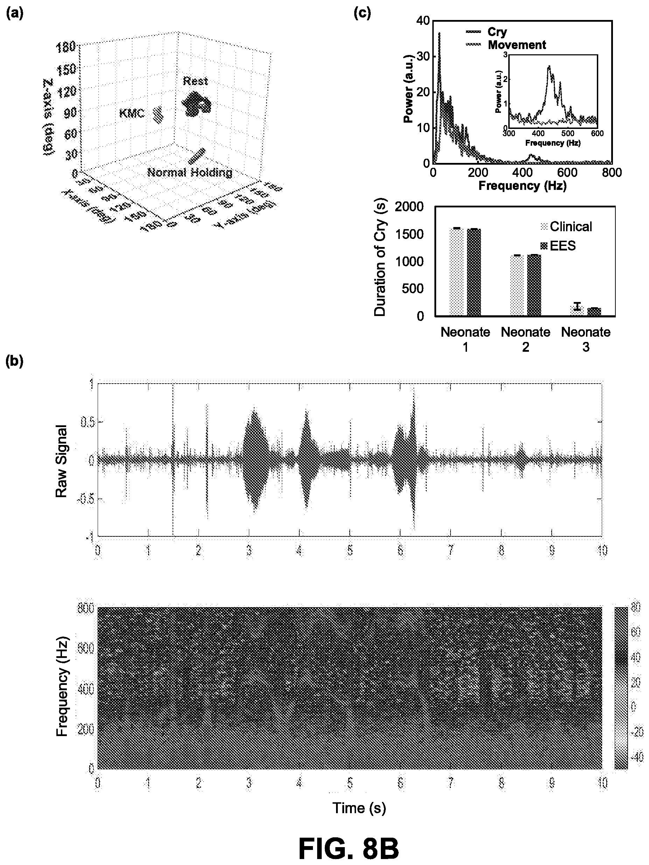

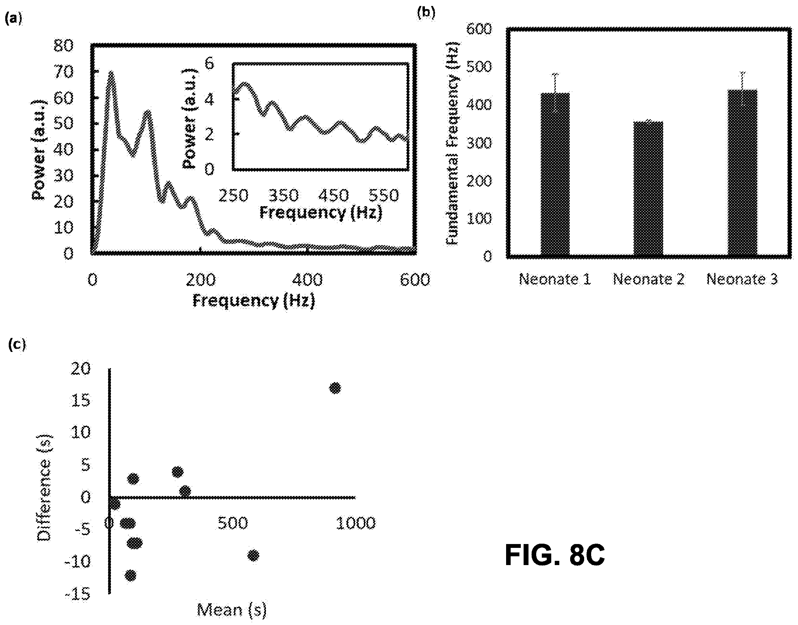

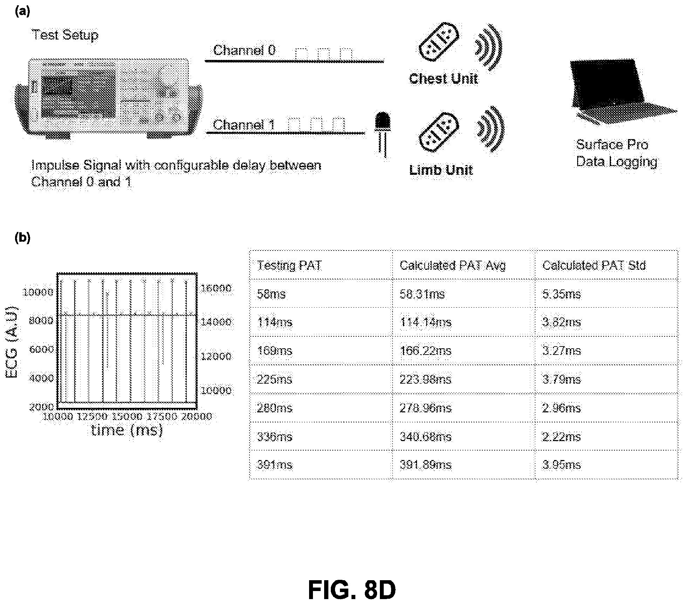

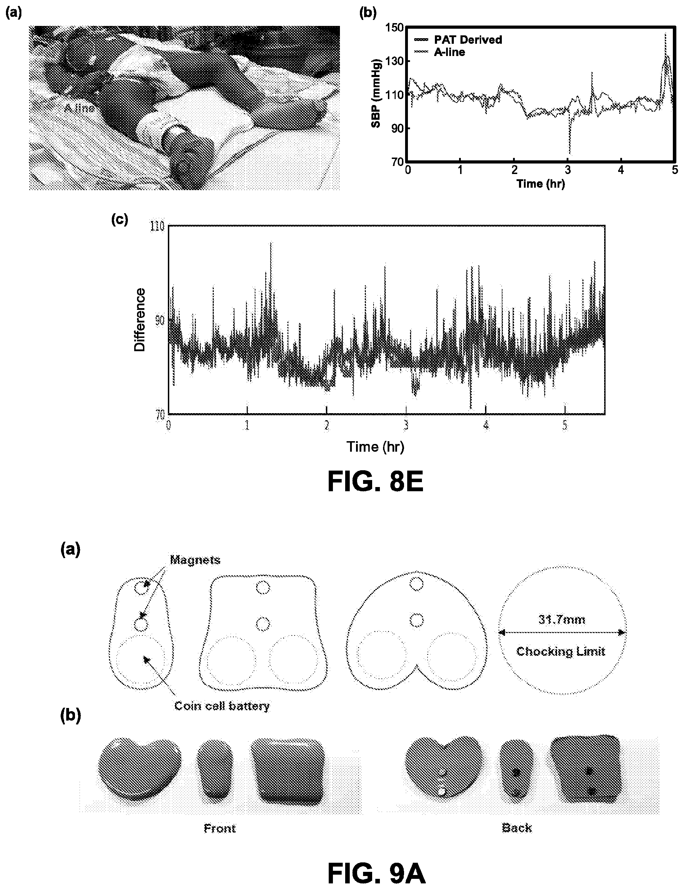

FIGS. 8A-8D shows advanced functionalities for neonatal/pediatric care with EES in clinical setting according to certain embodiments of the disclosure. FIG. 8A shows Kangaroo mother care (KMC) tracking and vital sign monitoring, where panel (a) shows accelerometry signal on various neonatal doll positions, including resting in-bed positions such as supine and right lateral, as well as parent non-KMC holding and typical KMC positions, panel (b) shows neonatal orientation of various positions on neonates in NICU, relative to gravity vector, captured by EES (N = 3), and panel (c) shows core and peripheral temperature monitoring with EES before, during, and after KMC on a premature neonate (GA 31 w). FIG. 8B shows cry signal analysis of neonatal patients, where panel (a) shows spectrogram of time-frequency signal from a neonate of GA 37 week, LGA (large-for-gestational-age) infant with feeding difficulties. Neonatal mechano-acoustic signal is presented from parent patting, neonatal crying, and resting events; panel (b) shows representative power spectrum of signal frequency upon fast Fourier transform processing of neonatal mechano-acoustic signal during crying and non-crying events; and panel (c) shows comparison of cry duration analysis between EES and human recording of individual neonates (N = 3) with a total of 11 cry events. FIG. 8C shows statistics of crying detection. FIG. 8D shows time synchronization validation, where panel (a) shows the schematic structure of the device, and panel (b) shows the validation data. FIG. 8E shows pulse arrival time (PAT) tracking from EES and its correlation with blood pressure on neonates, where panel (a) shows comparison between PAT-derived systolic blood pressure and blood pressure cuff (gold standard) during cycling trials on a healthy adult, panel (b) shows continuous neonatal blood pressure monitoring with EES (PAT-derived) and arterial line (A-line), and panel (c) shows PAT-derived blood pressure and its correlation with gold standard.
FIG. 9A shows removable battery sizes options for the EES according to certain embodiments of the disclosure, where panel (a) shows schematic layouts highlighting position of magnets and of one- or two-coin cell batteries, and comparison with the 31.7 mm diameter circle corresponding to choking hazard limit, and panel (b) shows photographic images of front side (left) and back side (right) of encapsulated batteries.

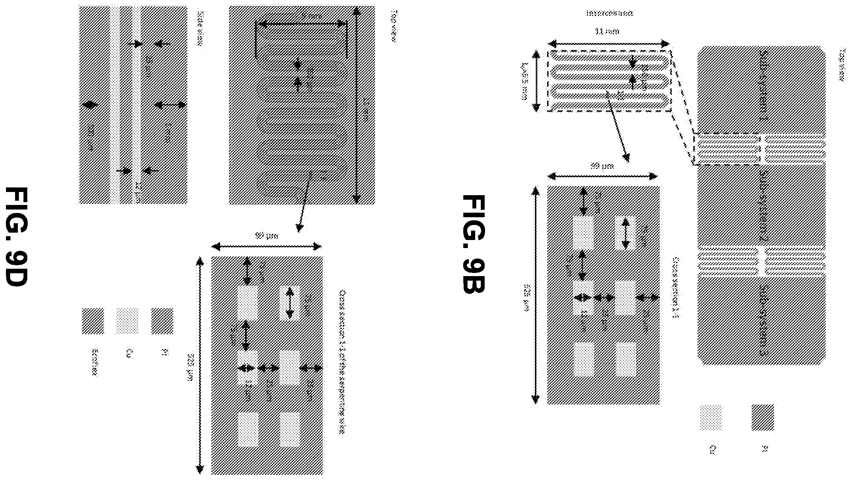

FIG. 9B shows schematic illustration of the serpentine interconnects used in a chest unit according to certain embodiments of the disclosure.

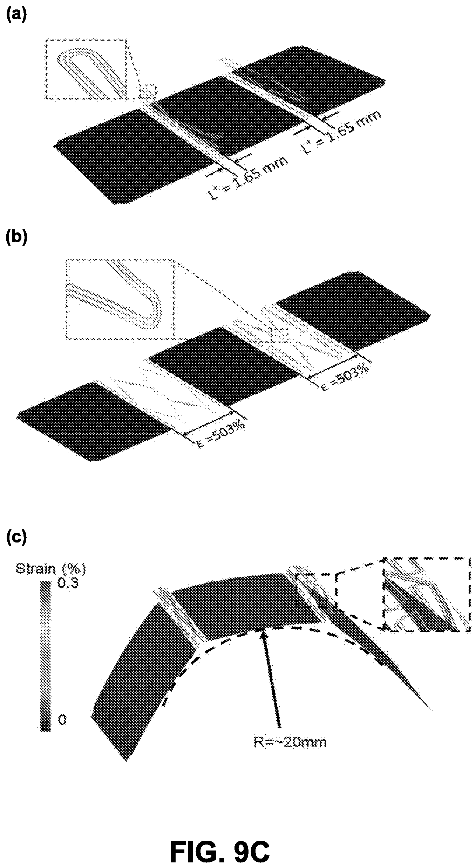

FIG. 9C shows computational demonstration of the mechanical properties of a chest unit according to certain embodiments of the disclosure, where panel (a) shows the initial length of interconnect (spacing between sub-systems) is L₀ = 5 mm, in which to increase the elastic stretchability, the interconnect is pre-compressed such that its initial horizontal length is reduced from L₀ = 5 mm mm to L* = 1.65 mm; panel (b) shows the simulation results from the finite element analysis (FEA) indicate that the elastic stretchability of the designed and optimized interconnects achieves 503%, where the elastic stretchability of the interconnects is defined as ε = (L-L*)/ L*, where L is the stretched length at which the copper layer in the interconnect yields; and panel (c) shows the simulation result of the strain in the copper layer of a chest unit for a bending radius of ~20 mm, where the equivalent bending stiffness of the chest unit is ~9.6 Nmm².
FIG. 9D schematically shows a representative interconnects used in the limb unit according to certain embodiments of the disclosure.

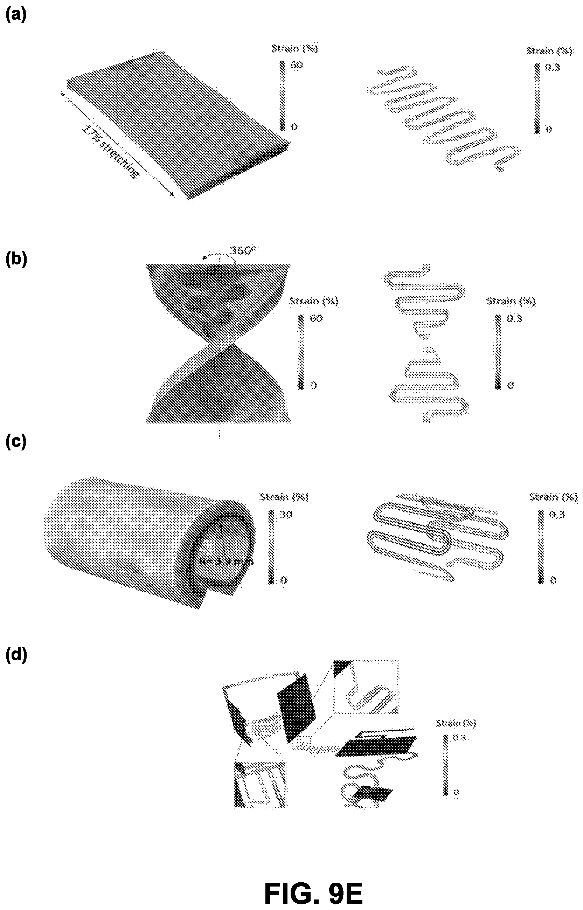

FIG. 9E schematically shows mechanical characteristics of a limb unit according to certain embodiments of the disclosure, where the strain distribution in the encapsulation layer (left) and copper layer (right) of a representative interconnect during panel (a) stretching, panel (b) twisting, panel (c) bending at the radius of 3.9 mm, and panel (d) the overall bending mechanics in a limb unit.

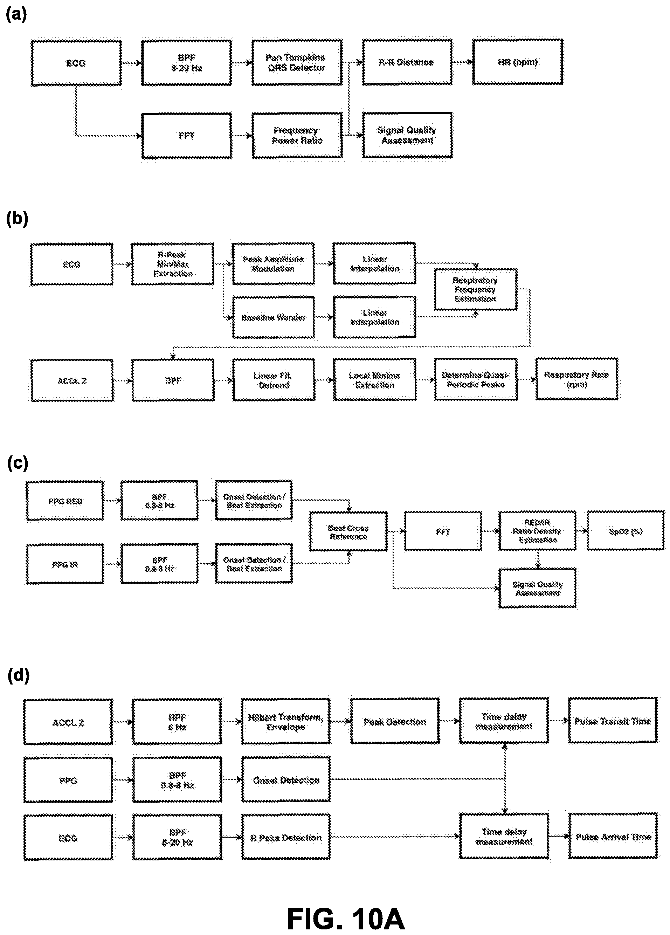

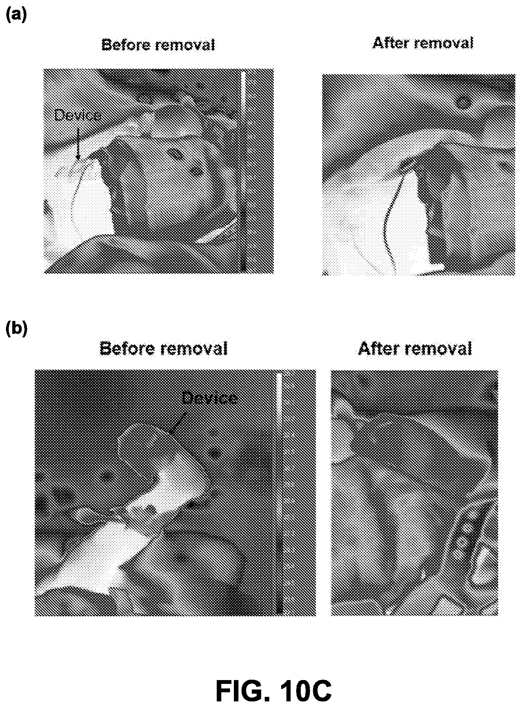

FIGS. 0A-10C shows data collection in the neonatal/pediatric intensive care units according to certain embodiments of the disclosure. FIG. 10A shows signal processing algorithms for panels (a) heart rate, (b) respiration rate, (c) blood oxygenation and (d) pulse arrival and transit time. FIG. 0B shows detailed signal processing algorithm for SpO₂, where processing of SpO₂ calculation algorithm for the signal are shown in panels (a) without motion artifact and (b) with motion artifact. FIG. 10C shows representative figures for safety related to heat generation of the device during an 24-hour operation, where panel (a) shows a chest unit did not create any significant heating after 24-hr operation, and panel (b) shows a limb unit did not create any significant heating after 24-hr operation.

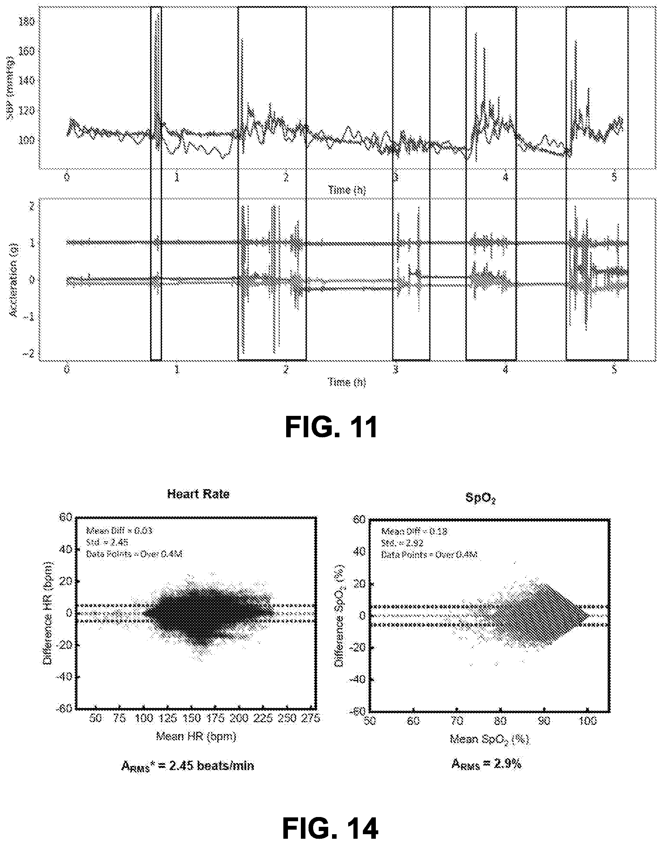

FIG. 11 shows schematic diagrams for capturing the events with motion artifact by the accelerometry data in a chest unit according to certain embodiments of the disclosure, where observation of larger movement in accelerometry data suggests that the spikes in SBP measured by A-line (red color) has a direct effect from motion of a subject.

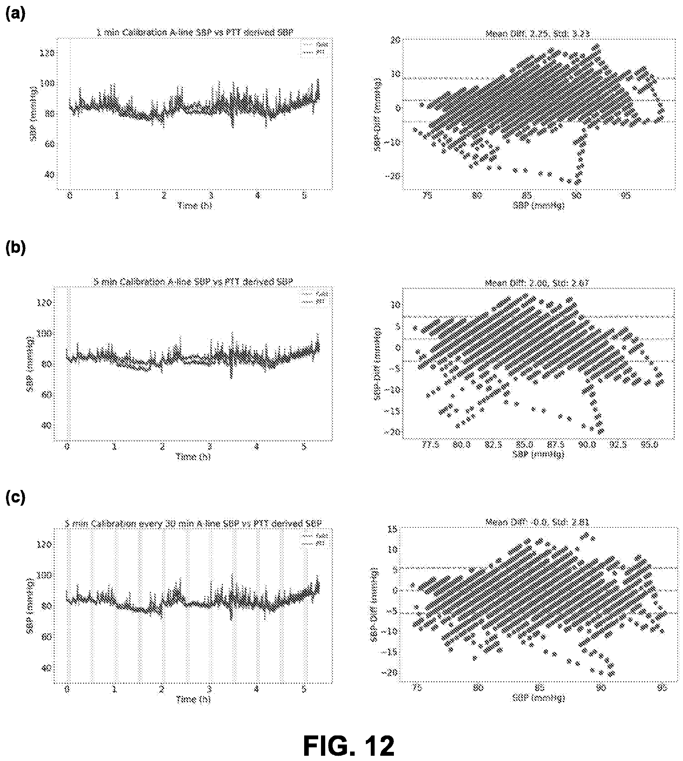

FIG. 12 shows the effect of calibration of window size and re-calibration interval according to certain embodiments of the disclosure, where panel (a) shows single calibration takes place with the initial one minute and panel (b) shows five minutes of PT data against A-line, and panel (c) shows another calibration scheme involves with re-calibration at every 30 minutes with the duration of 5 minutes of data. Longer duration of calibration shows the improvement both in mean difference and standard deviation. Re-calibration shows the effect in reducing mean difference.

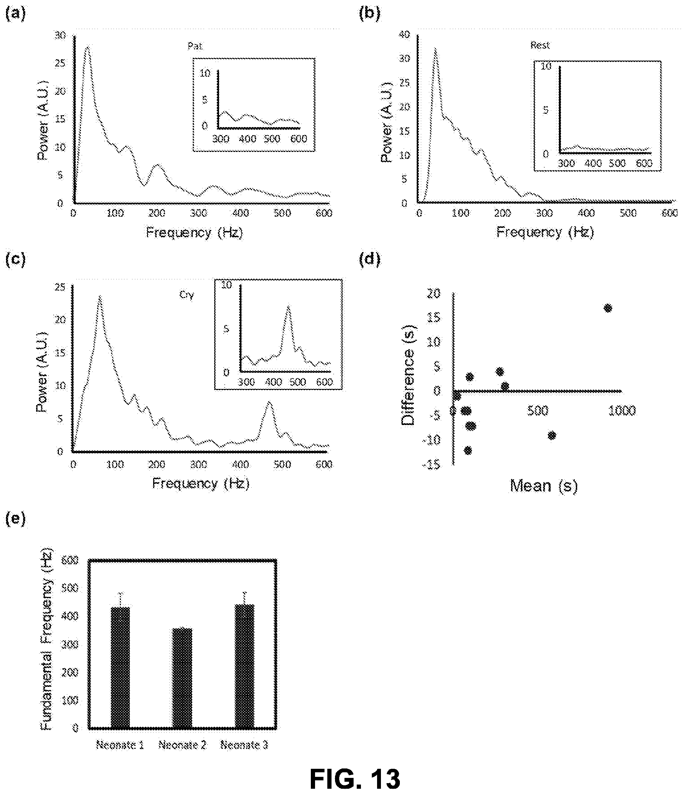

FIG. 13 shows cry characteristics captured by a chest unit in NICU according to certain embodiments of the disclosure, where panels (a)-(c) show representative power spectrum of signal frequency upon fast Fourier transform processing of neonatal mechano-acoustic signal during crying and non-crying events from a neonate in NICU. Neonatal mechano-acoustic signal is presented from (a) parent patting, (b) resting events, and (c) neonatal crying; panel (d) shows comparison of cry duration analysis between a chest unit and human recording of individual cry events; and panel (e) shows fundamental frequency of cry from each neonate (n = 3).
FIG. 14 shows a global BA plot for heart rate and blood oxygenation obtained in the all population (over 0.4 M data points) according to certain embodiments of the disclosure.

### DETAILED DESCRIPTION OF THE INVENTION

The present invention will now be described more fully hereinafter with reference to the accompanying drawings, in which exemplary embodiments of the present invention are shown. The present invention may, however, be embodied in many different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting and/or capital letters has no influence on the scope and meaning of a term; the scope and meaning of a term are the same, in the same context, whether or not it is highlighted and/or in capital letters. It will be appreciated that the same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification, including examples of any terms discussed herein, is illustrative only and in no way limits the scope and meaning of the invention or of any exemplified term. Likewise, the invention is not limited to various embodiments given in this specification.

It will be understood that, although the terms first, second, third, etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below can be termed a second element, component, region, layer or section without departing from the teachings of the present invention.

It will be understood that, as used in the description herein and throughout the claims that follow, the meaning of "a", "an", and "the" includes plural reference unless the context clearly dictates otherwise. Also, it will be understood that when an element is referred to as being "on," "attached" to, "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" to another feature may have portions that overlap or underlie the adjacent feature.

It will be further understood that the terms "comprises" and/or "comprising," or "includes" and/or "including" or "has" and/or "having" when used in this specification specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the figures. It will be understood that relative terms are intended to encompass different orientations of the device in addition to the orientation shown in the figures. For example, if the device in one of the figures is turned over, elements described as being on the "lower" side of other elements would then be oriented on the "upper" sides of the other elements. The exemplary term "lower" can, therefore, encompass both an orientation of lower and upper, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

As used in this disclosure, "around", "about", "approximately" or "substantially" shall generally mean within 20 percent, preferably within 10 percent, and more preferably within 5 percent of a given value or range. Numerical quantities given herein are approximate, meaning that the term "around", "about", "approximately" or "substantially" can be inferred if not expressly stated.

As used in this disclosure, the phrase "at least one of A, B, and C" should be construed to mean a logical (A or B or C), using a non-exclusive logical OR. As used herein, the term "and/or" includes any and all combinations of one or more of the associated listed items.

As used in this disclosure, the term "spatially separated" refers to two different locations on skin, where the two sensor systems disposed on those locations are not in physical contact. For example, one sensor system may be on the torso, and another sensor system on the limb.

As used in this disclosure, the term "mammal subject" refers to a living human subject or a living non-human subject. For the purpose of illustration of the invention, the apparatus and method are applied to monitor and/or measure physiological parameters of neonates or infants. It should be appreciated to one skilled in the art that the apparatus can also be applied to monitor and/or measure physiological parameters of children or adults in practice the invention.

The description below is merely illustrative in nature and is in no way intended to limit the invention, its application, or uses. The broad teachings of the invention can be implemented in a variety of forms. Therefore, while this invention includes particular examples, the true scope of the invention should not be so limited since other modifications will become apparent upon a study of the drawings, the specification, and the following claims. For purposes of clarity, the same reference numbers will be used in the drawings to identify similar elements. It should be understood that one or more steps within a method may be executed in different order (or concurrently) without altering the principles of the invention.

The ability to collect multimodal continuous vital signs that is time synced to each other provides deep insights on physiology. This has direct applications in healthcare monitoring. But, more specifically, this technology has direct utility in clinical trials research where physiological vital signs is an important endpoint to determine both the safety and efficacy of a new medication. This is specifically relevant for any medication that leads to a demonstrable change in any of the following physiological vital signs measured by this disclosure. This includes: heart rate, heart rate variability, stroke volume, chest wall displacement, ECG, respiratory rate, respiratory sounds (e.g. wheezing), blood oxygenation, arterial tone, temperature (both central and peripheral), cough count, swallowing, motion, sleep, and vocalization.

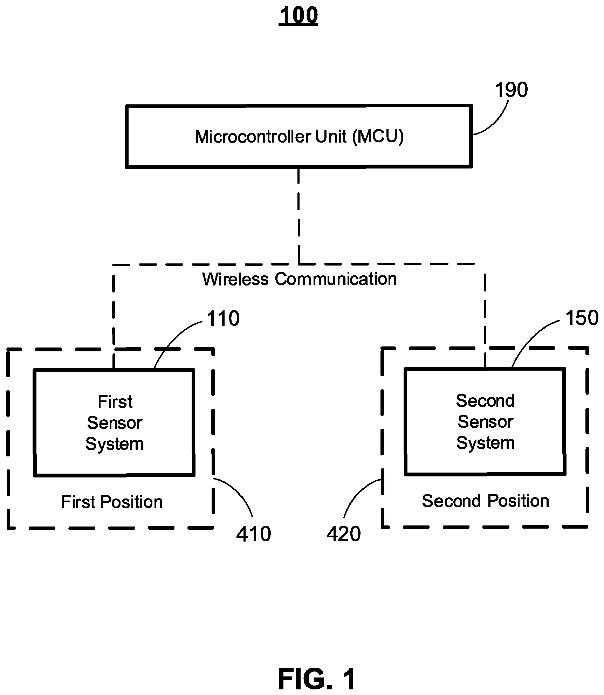

In one aspect, the disclosure relates to an apparatus for non-invasively and continuously measuring physiological parameters of a mammal subject. FIG. 1 schematically shows a functional block diagram of an apparatus according to certain embodiments of the present disclosure. As shown in FIG. 1, the apparatus 100 includes a plurality of sensor systems 110 and 150, namely a first sensor system 110 and a second sensor system 150, and a microcontroller unit (MCU) 190 adapted in wireless communication with the sensor systems 110 and 150. The sensor systems 110 and 150 are time-synchronized and communicate with each other wirelessly and bidirectionally, and are respectively attached to the mammal subject. In certain embodiments, each of the sensor systems is an epidermal electronic system (EES). For example, FIG. 1 shows that the first sensor system 110 is attached to a first position 410 of the mammal subject for detecting a first signal of the mammal subject, and the second sensor system 150 is attached to a second position 420 of the mammal subject for detecting a second signal of the mammal subject. In certain embodiments, the second position 420 is more distal or proximal to a heart of the mammal subject than the first position 410. For example, in one exemplary embodiment, the first position 410 is located at a torso region of the mammal subject, and the second position 420 is located at an extremity region or a limb region of the mammal subject. In this case, the first signal may be a heartbeat signal measured from the torso region, and the second signal may be a pulse signal measured from the extremity region or the limb region. In other words, the first sensor system 110 is a torso sensor system, and the second sensor system 150 is a limb sensor system. In other embodiments, the first position 410 and the second position 420 may be located at different regions of the mammal subject, as long as the first position 410 and the second position 420 are spatially separated. In certain embodiments, the first sensor system 110 can be an electrocardiography (ECG) sensor system, and the second sensor system 120 can be a photoplethysmography (PPG) sensor system. In certain embodiments, the first sensor system 110 and the second sensor system 150 can be implemented as separate physical devices. Alternatively, in certain embodiments, the first sensor system 110 and the second sensor system 150 can reside in a single physical device integrally.

Each of the sensor systems 110 and 150 includes one or more sensors that are used to detect a vital sign of the mammal subject, and then to generate one or more corresponding physiological parameters. In certain embodiments, the sensors may be various types of sensors for detecting the vital sign as a signal, and the signal can be, for example, an electrical signal related to at least one of electrocardiography (ECG) and electromyography (EMG) technology; a mechanical signal related to movement, respiration and arterial tonometry; an acoustic signal related to vocal cord vocalization and heart sound; and an optical signal related to blood oxygenation. The MCU 190 is configured to receive, from the sensor systems 110 and 150, output signals representing the physiological parameters, and to display the physiological parameters of the mammal subject. In certain embodiments, the MCU 190 may further process the output signals to obtain a specific vital sign of the mammal subject.

As discussed above, in certain embodiments, each of the sensor systems can be an EES. In certain embodiments, the first EES 110 can be an electrocardiography (ECG) EES, and the second EES 150 can be a photoplethysmography (PPG) EES. In certain embodiments, the first sensor system 110 is an ECG EES 110 (which is a torso sensor system), and the second sensor system 150 is a PPG EES 150 (which is a limb sensor system or an extremity sensor system).

FIGS. 2A-2D show schematic illustrations and photographic images of ultra-thin, skin-like wireless modules in the apparatus for measuring the physiological parameters in the neonatal intensive care unit (NICU) with comparisons to clinical standard instrumentation, according to embodiments of the disclosure. Specifically, FIGS. 2A and 2B shows functional block diagrams of the EES in two different exemplary embodiments.

Referring to FIG. 2A, in certain embodiments, the sensor member 123 includes, but is not limited to, two electrodes 121 and 122 spatially separated from each other by an electrode distance, D, for ECG generation. The electrodes 121 and 122 can be either mesh electrodes or solid electrodes. Further, the sensor member 123 also includes, but is not limited to, an instrumentation amplifier (e.g., Inst. Amp) electrically coupled to the two electrodes 121 and 122, adapted for eliminating the need for input impedance matching and thus making the amplifier particularly suitable for use in measurement and test equipment, and an anti-aliasing filter (AAF) electrically couple to the instrumentation amplifier and used before a signal sampler to restrict the bandwidth of a signal to approximately or completely satisfy the Nyquist-Shannon sampling theorem over the band of interest.

Further referring to FIG. 2A, the SoC 124 of the torso sensor system 110 includes, but is not limited to, a microprocessor unit, e.g., CPU, a near-field communication (NFC) interface, e.g., NFC ISO 15693 interface, general-purpose input/output (GPIO) ports, one or more temperature sensors (Temp. sensor), and analog-to-digital converters (ADCs) in communication with each other, for receiving data from the sensor member 123 and processing the received data.

Also referring to FIG. 2A, the transceiver 125 of the torso sensor system 110 is coupled to the SoC 124 for wireless data transmission and wireless power harvesting. In the exemplary embodiment, the transceiver 125 includes a magnetic loop antenna tuned to compliance with the NFC protocol and configured to allow simultaneous wireless data transmission and wireless power harvesting through a single link.

As shown in FIG. 2A, the sensor member 163 of the extremity sensor system 150 includes a PPG sensor located within a sensor footprint, which has an optical source having an infrared (IR) light emitting diode (LED) 161 and a red LED 162, and an optical detector (PD) electrically coupled to the IR LED 161 and the red LED 162. The sensor member 163 also includes, but is not limited to, an LED driver electrically coupled to the two electrodes 161 and 162 for driving the IR LED 161 and the red LED 162, and a trans Z amplifier electrically coupled to the PD.

Referring to FIG. 2A, the SoC 164 of the extremity sensor system 150 includes, but is not limited to, a microprocessor unit, e.g., CPU, a near-field communication (NFC) interface, e.g., NFC ISO 15693 interface, general-purpose input/output (GPIO) ports, one or more temperature sensors (Temp. sensor), and analog-to-digital converters (ADCs) in communication with each other, for receiving data from the sensor member 163 and processing the received data.

Still referring to FIG. 2B, the transceiver 165 is coupled to the SoC 164 for wireless data transmission and wireless power harvesting. In the exemplary embodiment, the transceiver 165 includes a loop antenna tuned to compliance with the NFC protocol and configured to allow simultaneous wireless data transmission and wireless power harvesting through a single link.

In addition, each of the plurality of spatially separated sensor systems further includes a plurality of flexible and stretchable interconnects (FIGS. 2C-2D) electrically connecting to a plurality of electronic components including the sensor member, the SoC and the transceiver; and an elastomeric encapsulation layer (FIGS. 2C-2D) surrounding the electronic components and the plurality of flexible and stretchable interconnects to form a tissue-facing surface and an environment-facing surface, wherein the tissue-facing surface is configured to conform to a skin surface of the mammal subject. In one embodiment, the encapsulation layer includes a flame retardant material.

In operation, the torso sensor system 110 (ECG EES 110) and the extremity sensor system 150 (PPG EES 150) are in wireless communication with a reader system 190, alternatively, a microcontroller unit (MCU), having an antenna 195. Specifically, the RF loop antennas 125 and 165 in both the torso sensor system 110 (ECG EES 110) and the extremity sensor system 150 (PPG EES 150) are in wireless communication with the antenna 195 and serve dual purposes in power transfer and in data communication, as shown in FIG. 2A. In one embodiment, the reader system 190 also includes, but is not limited to, an NFC ISO 15693 reader, a circular buffer and a Bluetooth Low Energy (BLE) interface, which are configured such that data can be continuously streamed at rates of up to 800 bytes/s with dual channels, which is orders of magnitude higher than those previously achieved in NFC sensors. A key to realizing such high rates is in minimizing the overhead associated with transfer by packaging data into 6 blocks (24 Bytes) in the circular buffer. The primary antenna 195 connects to the host system for simultaneous transfer of RF power to the ECG EES 110 and the PPG EES 150. As such, the apparatus can operate at vertical distances of up to 25 cm, through biological tissues, bedding, blankets, padded mattresses, wires, sensors and other materials found in NICU incubators, for full coverage wireless operation in a typical incubator. BLE radio transmission then allows transfer of data to a personal computer, tablet computer or smartphone with a range of up to 20 m. Connections to central monitoring systems in the hospital can then be established in a straightforward manner.

In another embodiment as shown in FIGS. 2B-2D, the first sensor system 210 and the second sensor system 250 are similar to the first sensor system 110 and the second sensor system 150 shown in FIG. 2A, except that each of the first sensor system 210 and the second sensor system 250 further includes a battery 205 for provide power to said sensor system, and a power management unit/IC (PMIC) 206 electrically coupled with the battery 205, the SoC 224/264 and the transceiver (antenna) 195. The power management unit 206 operably involves dual power operation mode from primary wireless power transfer and the secondary battery 205 for portability. In addition, the sensor member (or sensor circuit) 223 of the first sensor system (ECG EES) 210 also includes optional electrode for fECG measurement and 6 axial inertial measurement unit (IMU) for seismocardiography (SCG) and respiratory rate measurement on the top of an ECG analog front end (AFE). The sensor member (or sensor circuit) 263 of the second sensor system (PPG EES) 250 also includes also a PPG AFE and 6 axial IMU for motion artifact reduction algorithm. The SoC 224/264 of each of the first sensor system 210 and the second sensor system 250 further includes a down-sampler and BLE radio. Each of the power management unit 206 and the sensor members 223 and 263 is controlled by BLE SoC 224/264.

In certain embodiments, the battery 205 is a rechargeable battery operably recharged with wireless recharging. In one embodiment, the electronic components of each of the first sensor system 210 and the second sensor system 250 further include a failure prevention element that is a short-circuit protection component or a battery circuit (not shown) to avoid battery explosion.

In the embodiments as discussed above, each of the sensor systems can be an EES. However, in certain embodiments, one or more of the sensor systems may be a system other than the EES. For example, in one embodiment, the first sensor system 110 as shown in FIG. 1 may be implemented as an inertial motion sensor system or an accelerometer system, and the second sensor system 110 may still be a PPG EES.

FIG. 3A shows a flowchart of a method of non-invasively measuring physiological parameters of a mammal subject according to certain embodiments of the present disclosure. In certain embodiments, the method as shown in FIG. 3A may be implemented on the apparatus as shown in FIG. 1. It should be particularly noted that, unless otherwise stated in the disclosure, the steps of the method may be arranged in a different sequential order, and are thus not limited to the sequential order as shown in FIG. 3A.

As shown in FIG. 3A, at procedure 310, the sensor systems (i.e., the first sensor system 110 and the second sensor system 150 as shown in FIG. 1) are utilized with the mammal subject. For example, the first sensor system 110 is attached to a first position in the torso region 410 of the mammal subject for measuring a heartbeat of the mammal subject, and the second sensor system 150 is attached to a second position in the limb region 420 of the mammal subject for measuring a pulse of the mammal subject. Further, the sensor systems 110 and 150 are in wireless communication with the MCU 190, and are time-synchronized and spatially separated by a distance defined by the first and second positions.

At procedure 320, the sensor systems 110 and 150 are used to measure or monitor the physiological parameters of the mammal subject. In certain embodiments, the physiological parameters of the mammal subject may include one or more of: heart rate, heart rate variability, heart sounds, blood pressure, chest wall displacement, electromyography, electrocardiography, blood oxygenation, respiratory rate, respiratory effort, respiratory cadence, tidal volume, coughing, snoring, sneezing, throat clearing, wheezing, apnea, hypoapnea, physical activity, core body position, peripheral limb position, scratching, vocalizations, rubbing, walking, sleep quality, sleep time, wake time upon sleeping, skin temperature, core body temperature, and a combination thereof. Once the physiological parameters are obtained, the sensor systems 110 and 150 may respectively generate corresponding output signals, which are then transmitted wirelessly to the MCU 190.

At procedure 330, the MCU 190 receives the physiological parameters from the sensor systems 110 and 150. Specifically, the MCU 190 receives the output signals from the first sensor system 110 and the second sensor system 150, and then processes the output signals to obtain the physiological parameters. At procedure 340, the MCU 190 may display the physiological parameters.

As discussed above, one of the physiological parameters that may be monitored or measured is the blood pressure of the mammal subject. FIG. 3B shows a flowchart of a method of non-invasively and continuously measuring blood pressure of a mammal subject according to certain embodiments of the present disclosure. In certain embodiments, the method as shown in FIG. 3B may be implemented on the apparatus as shown in FIG. 1. It should be particularly noted that, unless otherwise stated in the disclosure, the steps of the method may be arranged in a different sequential order, and are thus not limited to the sequential order as shown in FIG. 3B.

As shown in FIG. 3B, at procedure 350, the MCU 190 receives the output signals from the first sensor system 110, which is disposed in a first position 410 of the mammal subject for measuring a first signal of the mammal subject, and the second sensor system 150, which is disposed in a second position 420 of the mammal subject for measuring a second signal of the mammal subject. In certain embodiments, the first position 410 is at a torso region of the mammal subject, and the second position 420 is at an extremity region or a limb region of the mammal subject. In this case, the first signal may be a heartbeat signal detected from the torso region, and the second signal may be a pulse signal detected from the extremity region or the limb region. At procedure 355, the MCU 190 may process the output signals to determine a pulse arrival time (PAT) as a time delay Δt between detection of the first signal and detection of the second signal. Once the PAT is determined, at procedure 360, the MCU 190 may then determine a pulse wave velocity (PWV) based on the PAT and a pulse arrival distance L between the first position 410 and the second position 420. In one embodiment, the PWV is determined by: $PWV = \frac{L}{Δ t}$ (1)

Once the PWV is obtained based on equation 1, at procedure 365, the MCU 190 may further calculate and determine the blood pressure P of the mammal subject from the PWV, where P is a parabolic function of the PWV. In one embodiment, the relation between P and PWV can be represented by: $P = {αPWV}^{2} + β ,$ (2) where α and β are empirically determined constants depending on artery geometry and artery material properties of the mammal subject. In one embodiment, at a blood pressure range between 5 kPA and 20 kPa, $0.13 kPa \times {s}^{2} / {m}^{2} \leq α \leq 0.23 kPa \times {s}^{2} / {m}^{2} ;$ and $2.2 kPa \leq β \leq 3.2 kPa .$

In one embodiment, each of the sensor systems further includes a power supply, and the power supply is an embedded power supply or a detachable modular power supply.

In one embodiment, each of the sensor systems is in wireless communication with the MCU via a near field communication (NFC) protocol, or Bluetooth protocol. In one embodiment, each of the sensor systems includes a magnetic coil in compliance with the NFC protocol to allow wireless data transmission and wireless power transmission through a single link.

In one embodiment, each of the sensor systems further includes one or more of: an accelerometer for position or movement monitoring; and a temperature sensor for measuring temperature.

In one embodiment, each of the sensor systems is waterproof.

In certain embodiments, the sensor systems, apparatus and method as discussed above are versatile and may be used for a variety healthcare application including clinical applications such as:
- Critical care monitoring in neonatal intensive care units
- Critical care monitoring in pediatric intensive care units
- Critical care monitoring in neonatal / pediatric cardiac care units
- Critical care monitoring in neonatal / pediatric neurocritical care units
- Post-discharge home monitoring for high-risk neonates
- Ante-partum home monitoring for high risk maternal / fetal monitoring
- Intra-partum monitoring for laboring women
- Post-partum monitoring for high-risk women
- Digital health / digital medicine
- Clinical trials

In certain embodiments, the sensor systems and apparatus as discussed above may further be used as comprehensive, continuous, and on-body sensor systems in the support and development of therapeutic agents that affect physiological parameters. Clinical trials remain an expensive, high-risk proposition for new medicines. There is a constant need for new outcome measurement tools that detect and measure small, but clinically meaningful changes. These tools serve several purposes:
- Provide an objective indication of the benefit and risk of any given medication in both a critical care, acute, outpatient, or home setting
- Serve as regulatory endpoints facilitating the approval of a new therapeutic by regulatory agencies (e.g. FDA)
- Post-marketing surveillance of a drug's safety and efficacy
- Data that supports prescribing label information and marketing
- Engagement of patients within a digital ecosystem to track physiological outcomes, solicit patient-reported outcomes, and provide real time or summary feedback to both the user and clinical provider

In certain embodiments, the apparatus and method as discussed above may be used in a variety of different applications. For example, the applicability of the technology is broad across a wide range therapeutic agents. Any agent that affects physiological vital signs characterized as electrical signals (e.g. ECG, EMG), mechanical signals (e.g. chest wall movement, respiration, arterial tonometry), acoustic signals (e.g. vocal cord vocalization, heart sounds), and optical signals (e.g. blood oxygenation) would be applicable to pair with the technology described herein.

In certain embodiments, there are therapeutics to pair with this technology that hold the greatest relevance given the direct impact on measureable physiological parameters that the sensors measure. Specifically, therapeutics that are used in critical care, infectious disease, pulmonology, and cardiology are most relevant.

In certain embodiments, the apparatus and method as discussed above are also applicable to diagnosis, monitoring, management and treatment in the context of applications for infectious diseases:
- Developmental vaccines and therapeutics for RSV (respiratory syncytial virus) infections: examples include RSV 6120/ΔNS1, RSV 6120/F1/G2/ΔNS1, RSV-MVA-BN^{®} Vaccine, RSV F Vaccine, ALS-008176, BTA-C585, PC786, ALN-RSV01, PanAd3-RSV, MVA-RSV, Presatovir, GSK3844766A, GSK3389245A, GSK3003891A, GSK3888550A, AK0529, Heliox, Palivizumab, GS-5806, MDI8897, PC786, EDP-938, RSV ΔNS2/Δ1313/I1314L, RSV 276, RSV D46/NS2/N/ΔM2-2-HindIII, D46/NS2/N/ΔM2-2-HindIII, RSV LID ΔM2-2 1030s, GSK3003892A, GSK3003893A, GSK3003895A, GSK3003896A, GSK3003898A, GSK3003899A, motavizumab (MEDI-524), presatovir, RSV cps2 Vaccine, RSV ΔNS2 Δ1313 I1314L Vaccine, virazole, A-60444, GS-5806, suptavumab, RV521, AK0529, RSV polyclonal immunoglobulin, VRC-RSVRGP084-00VP, Ad26.RSV.preF, RSV A Memphis 37, Influenza A/California/04/2009, rRSV A/Maryland/001/11, Ad35.RSV.FA2, DPX-RSV(A), RSV(A)-Alum, JNJ-53718678, ALS-008176, BTA9881, MEDI8897, lumicitabine, ALX-0171, EDP-938, SeVRSV vaccine, EDP-938, rBCG-N-hRSV 1/100, danirixin, CXCL1, ALS-008176, MDT-637, ALS-008176, ALS-008112, PC786, Bexsero, GSK3389245A_HD, RSV preF Protein, JNJ-64417184, adenovirus RSV-TK, and MDT-637, GSK3389245A, MEDI8897, MVA-BN RSV, rBCG-N-hRSV, synGEM, VXA-RSV-f oral, SeVRSV, RSV 6120/delta NS2/1030s
- Developmental vaccines and therapeutics for Ebola virus: examples include BCX4430, brincidofovir, favipiravir, GS-5734
- Developmental vaccines and therapeutics for tuberculosis: examples include sutezolid, BTZ 043, nitazoxanie, Q203, SQ109, Ad5 Ag85A, DAR-901, H1:IC31, H4:IC31, H56: IC31, ID93+GLA-SE, M72+AS01E, MTBVAC, RUTI, TB/FLU-04L, vaccae, VPM 1002 (rBCG), bedaquiline, delpazolid, GSK-3036656, OPC-167832, PBTZ-169, Q203, SQ109, sutezolid, TBA-7371
- Developmental vaccines and therapeutics for zika virus: butantan attenuated, Butantan ZIKV, ChadOx1-Zk, Chimerivax-Zika, GEO-ZM05, GLS-500, mRNA-1325, MV-Zika, NI.LV-Zk, replikins zika vaccine, rZIKV-3'D4delta30
- Developmental vaccines and therapeutics for malaria: ACT451840, AQ13, artefenomel, artemisinin-napthoquine, artesunate+ferroquine, CDRI 87/78, DSM265, fosmidomycin, KAE609, KAF156 / lumefantrine, MMV390048, P218, SAR97276, sevuparin, SJ557733, tafenoquine
- Developmental vaccines and therapeutics for dengue: CYD-TDV, TDENV-PIV, TDENV-LAV
- TDV,TV003,TVDV, V180
- Developmental vaccines and therapeutics for rift valley rever: examples include RVP MP-12
- Developmental vaccines and therapeutics for pneumococcal infections
- Developmental vaccines and therapeutics that are antibiotics, antivirals, antifungals, or anti-parasitic used in the context of sepsis or critical care
- Developmental vaccines and therapeutics for influenza
- Developmental vaccines and therapeutics for pneumonia (bacterial, fungal, and viral)

In certain embodiments, the apparatus and method as discussed above are also applicable to diagnosis, monitoring, management and treatment in the context of sleep medicine:
- Developmental vaccines and therapeutics for obstructive sleep apneas: example include therapeutics that target noraderaneline and dopamine, therapeutics that target potassium channel blockers, therapeutics that modulate serotonin, therapeutics that target acetylcholine, tetrahydrocannabinols, xanthines, carbonic anhydrase inhibitors, and drugs that target γ-aminobutyric acid-benzodiazepine receptor complexes.

In certain embodiments, the apparatus and method as discussed above are also applicable to diagnosis, monitoring, management and treatment of applications involving cardiology:
- Developmental vaccines and therapeutics for cardiac arrhythmias
- Developmental vaccines and therapeutics that affect blood pressure (both pressors and anti-hypertensives)

In certain embodiments, the apparatus and method as discussed above are also applicable to diagnosis, monitoring, management and treatment of applications involving respiratory medicine:
- Developmental vaccines and therapeutics for asthma
- Developmental vaccines and therapeutics for chronic obstructive pulmonary diseases
- Developmental vaccines and therapeutics for infant respiratory distress syndrome
- Developmental vaccines and therapeutics for cystic fibrosis

In certain embodiments, the apparatus and method as discussed above are also applicable to diagnosis, monitoring, management and treatment of applications involving allergy / immunology:
- Developmental vaccines and therapeutics for allergic diseases including anaphylaxis
- Developmental vaccines and therapeutics for allergic diseases that affect the lungs

FIGS. 3C-3E show a plurality of flowchart of different applications of the apparatus and method as discussed above according to certain embodiments of the present disclosure. In certain embodiments, the applications and methods as shown in FIGS. 3C-3E may be implemented on the apparatus as shown in FIG. 1. It should be particularly noted that, unless otherwise stated in the disclosure, the steps of the methods may be arranged in a different sequential order, and are thus not limited to the sequential order as shown in each of FIG. 3C-3E.

FIG. 3C shows a flowchart of a method developing vaccines for a disease on a mammal subject according to certain embodiments of the present disclosure. As shown in FIG. 3C, at procedure 370, a vaccine agent is provided to the mammal subject not having the disease. Once the vaccine agent is provided, at procedure 372, the mammal subject is monitored, continuously for a period of time, to obtain physiological parameters of the mammal subject. At procedure 375, the effects of the vaccine agent on the mammal subject in the period of time can be evaluated based on the physiological parameters obtained.

FIG. 3D shows a flowchart of a method for developing therapeutics for a disease on a mammal subject according to certain embodiments of the present disclosure. As shown in FIG. 3D, at procedure 380, a therapeutic agent is provided to the mammal subject having the disease. Once the therapeutic agent is provided, at procedure 382, the mammal subject is monitored, continuously for a period of time, to obtain physiological parameters of the mammal subject. At procedure 385, the effects of the therapeutic agent on the disease in the period of time can be evaluated based on the physiological parameters.

FIG. 3E shows a flowchart of a method for diagnosing a disease on a mammal subject according to certain embodiments of the present disclosure. As shown in FIG. 3E, at procedure 390, a mammal subject is monitored, continuously for a period of time, to obtain physiological parameters of the mammal subject. At procedure 392, a determination can be made as to whether the mammal subject has the disease based on the physiological parameters. In the case where the mammal subject is diagnosed to have the disease, at procedure 395, a corresponding treatment of the disease can be performed based on the physiological parameters. In one embodiment, the treatment includes providing a respiratory medicine to the mammal subject, where the type and dosage of respiratory medicine can be determined based on the physiological parameters.

The apparatus and methods as discussed above may be used in or as a part of a vital sign monitoring system and/or a pediatric medical devices. In certain embodiments, provided herein are battery-powered, wireless (e.g., Bluetooth 5 enabled) vital signs monitoring system that exploits a bi-nodal pair of thin, low-modulus measurement modules, capable of gently and non-invasively interfacing onto the skin of neonates, even at gestational ages that approach the limit of viability. A key distinguishing features of this technology includes low-battery power operation enabling at least 24-hour continuous use between charges while enabling monitoring of a full suite of vital signs. The designs enable measurement of traditional vital signs in addition to advanced physiological parameters not currently measured. The skin interface and electrical/mechanical design of the sensor allows for safe integration with fragile neonatal skin even during life-saving interventions such as cardiac defibrillation. The invention also include systems that are powered using wireless means such as using wireless energy harvesting approaches.

In certain embodiments, the methods as discussed above may use any of the sensor networks, sensor systems and electronic components described herein. In certain embodiments, the disclosure also relates to any sensor networks for carrying out any of the methods described herein.

In certain embodiments, the disclosure provides a sensor network for wireless monitoring of physiological parameters comprising: a plurality of time-synchronized sensor systems, wherein each sensor system comprises a sensor to measure or monitor a physiological parameter; a bidirectional wireless communication system for wirelessly transmitting data to and from the plurality of time-synchronized sensor systems; and a remote reader in communication with the bidirectional wireless communication system for real-time display of the monitored physiological parameters, recording of the monitored physiological parameters, and/or alarm for an out of agreement state.

In certain embodiments, the disclosure provides a wireless sensor system that is modular in nature allowing for a detachable power supply (e.g. battery). In an embodiment, the disclosure provides a wireless sensor system with waterproof functionality allowing for use in aquatic or highly humid conditions or high sweating. In an embodiment, the disclosure provides a wireless sensor system for use cases related to clinical trials research, support the approval of new therapeutics, and digital health.

In certain embodiments, features of the disclosure may include:
- Novel folded electronic board design to minimize surface area of the sensor enabled by serpentine interconnects
- Multimodal power options including a removable, modular battery fully decoupled from the electronic circuit board to allow for retention of the device on fragile skin but continued operation with a rapid battery replacement
- Longer operation times with lower power operation extending at least 72 hours
- Application of this technology towards assessing digital medicine - specifically, coupling the device as a drug development tool for clinical trials.

FIG. 4 shows a flow diagram illustrating use of wearable sensor technology to support supports the development, testing, approval, and post-market tracking of a wide range of therapeutic agents according to certain embodiments of the present disclosure.

In certain embodiments, the apparatus and methods as discussed above provide advantages relevant to a broad range of applications:
- Mechanics: The use of folded electronics boards as well as serpentine interconnects provides for high stretchability of the board with minimized surface area incorporating electronics components. Encapsulation of both ECG and PPG in a soft silicone shell results in no exposure of wires and electronics. More precisely, encapsulation process involve the use of a flat layer of silicone (examples include Silbione RTV 4420 from Elkem) on which electronics is laminated using an adhesive layer, and a shell layer of same silicone that covers this electronics. Flat layer and shell are bonded through an uncured silicone layer. Prior to encapsulation, a layer of soft silicone gel is also added on silicone board (examples include Silbione RT GEL 4717 or Ecoflex Gel) to enhance overall softness of device, acting as a strain insulation layer. Curing steps for all silicones are performed in oven (temperature range 70-100°C). Resulting devices combine skin compatibility to stretching capabilities at a level that matches usual deformations undergone when placed on body.
- Electrode water proofing: ECG device comprises two electrodes for capturing ECG electrical signal from the body. To provide a waterproof device, electrical contact at the two measurement points is made through carbon black PDMS (CB-PDMS) pads that are fully bonded (e.g. using Corona treatment) to the main silicone encapsulation shell, resulting in a silicone encapsulation with no openings. Contact from CB-PDMS pads to gold electrodes on the electronics board is made through conductive adhesive.
- Power: To allow for better modularity, design includes three different powering schemes: (i) embedded battery, (ii) modular battery, (iii) modular coil. For (ii) and (iii), battery and coil modules are interchangeable and connected with a main module (comprising all electronics except powering part) through magnets connections. Enabling a replacement of battery without detaching device from fragile skin allows for limited number of adhesive peeling events. In addition, the absence of battery in the main module makes this part autoclavable for wider sanitization practices compatibility. Interchangeable battery units include various lifetimes associated to capacity and size variations with the battery unit coupling to the magnet interconnects on the sensor board. The thinnest profile is achieved using a CR1216 coin cell battery, resulting in a battery module with a maximum thickness of 3mm.

In certain embodiments, the apparatus and methods as discussed above provide certain advantages over systems of the related art. Prior groups have developed neonatal vests with embedded sensors and wireless communication capabilities. Others have instrumented neonatal beds. These systems are impractical because they are bulky and cover a significant surface area of the neonate - which further complicates medical care instead of simplifying it. Another previously reported technology is only in the research phase - it still requires multiple wires and lacks the intimate skin connection that enables high fidelity sensing, particularly in the context of a neonate that is moving.

These and other aspects of the present invention are further described below. Without intent to limit the scope of the invention, examples according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action. The invention is defined in the appended claims.

### EXAMPLE 1

This example, related to one aspect of the disclosure, relates to a binodal, wireless, and mechanically soft electronic platform that monitors physiological signals continuously and noninvasively for up to 24 hours on neonatal and pediatric patients. Engineering advancements of this wearable platform include multimodal powering options, soft mechanics, and advanced clinical diagnostic functionalities that aim to enhance neonatal and pediatric patient care: quantification of therapeutic skin-to-skin care or called Kangaroo Mother Care (KMC), cry signal pattern and duration, and non-invasive, continuous blood pressure assessments, along with wireless capturing of clinical vital signs, including heart rate, respiration rate, temperature, and pulse oxygenation. The platform was validated by clinical studies with 40 neonatal patients in the neonatal intensive care unit (NICU) of 23-41 weeks gestational age (GA) and 10 pediatric patients in the pediatric intensive care unit (PICU) of up to 3 years in chronical age (CA). Clinical studies show that this platform demonstrates accurate vital sign measurements continuously for up to 24 hours when compared with clinical standards in the hospital, while reliably providing more advanced functionality beyond measuring vital signs such as tracking of kangaroo mother care and crying activities.

Specifically, this example demonstrates a neonate-friendly, soft and stretchable electronic platform, referred to as the EES, which would allow long-duration wireless monitoring of physiological signals for up to 24 hours. This platform was clinically validated in the neonatal/pediatric intensive care units, demonstrating long duration, accurate, non-invasive measurements of vital signs including heart rate (HR), respiration rate (RR), pulse oxygenation (SpO₂), temperature, and blood pressure (BP), when compared with clinical gold standards. Furthermore, the multimodal wireless devices enable exploration of physiological signals outside of conventional clinical standard, such as cry analysis and therapeutic skin-to-skin care tracking for the improvement of neonatal and pediatric care.

KMC is a therapeutic method where a newborn is held against a parent's chest to provide skin-to-skin contact. KMC is known to lower neonatal mortality, stabilizes heart rate, temperature, and respiration rate, and decreases the risk of infection. In low-resource countries, KMC is continuously performed in lieu of high-cost incubators to enhance neonatal health and parental/infant bonding. However, despite the therapeutic benefits of KMC, it remains difficult to quantify KMC compliance, often relying on self-reporting by the parent. In addition, vital sign monitoring during KMC sessions are especially challenging with involvement of wired sensors on neonates. A system having wireless mode of operation and mechanics that is non-obstructive to skin-to-skin contact, that can not only identify KMC event but also measuring vital signs concurrently, would therefore provide means to quantify the benefits of KMC and fruitful information to parents and caregivers consequently.

In this example, a mechanically soft and stretchable wireless electronic platform is provided for neonatal and pediatric vital sign monitoring validated with the continuous operation up to 24 hours. This platform provides multimodal power options that can be operated based on clinical and user preference: (1) embedded battery platform, where an in-sensor, rechargeable battery supports the electrical power required to operate the system, providing the advantage of long-term vital sign monitoring, stable operation, and cost-effectiveness, (2) replaceable battery platform, where power is provided through a battery interface that can be replaced without disturbing the skin/sensor interface, an option especially attractive when providing care for premature neonates with undeveloped skin, (3) wireless power transfer platform, where a modular unit with the RF loop antenna is powered by the primary antenna located underneath a typical incubator, there-in providing complete battery-free operation with the thinnest profile of the overall sensor. In this example, the inventors have validated the platform with 50 patients under 3 years old in the neonatal and pediatric intensive care units, and clinical characteristics of these neonates are listed in a Table as shown in FIG. 5. Quantitative validation of the full range of capabilities, with comparisons to gold standards, involve continuous monitoring for periods up to 24 hours are presented. The results show that this platform provides continuous, real-time vital sign monitoring for up to 24 hours, with no negative effects even on skin even for neonates having fragile skin, and high accuracy upon comparison with clinical gold standards. In addition, this platform sheds light onto the detection of non-standard physiological signals such as cry activity monitoring as an indicator of neonatal stress, and KMC tracking, providing a platform for novel insights to improve neonatal and pediatric care.

### Results

### Wireless Vital Signs Monitoring System Sensor Design

FIG. 6A schematically shows a functional block diagram of core components of an apparatus according to the present invention including two time-synchronized EES including analog-front-end for ECG processing 3-axis accelerometer, thermometer IC, and the BLE SoC for the Chest EES and pulse oximeter IC, thermometer, and the BLE SoC for the Limb EES. Specifically, the apparatus as shown in FIG. 6A includes a Chest EES and a Limb EES. Three different power sources supplying required power to operate for each EES. The Chest EES includes an ECG sensing unit, a motion sensing unit through a 3-axial accelerometer (BMI160, Bosch Sensortec), and a clinical-grade thermometer (MAX30205, Maxim Integrated). The ECG sensing unit includes two gold plated electrodes, an instrumentation amplifier, analog filters, and amplifiers, and a BLE SoC (nRF52832, Nordic Semiconductor). Remained for black PDMS. Data acquisition of the motion sensing by the accelerometer is controlled by BLE SoC through Serial Peripheral Interface (SPI) communication protocol, while the temperature data by the thermometer is acquired through the Inter-integrated Circuit (I2C) communication protocol. The Limb EES includes an integrated pulse oximetry module (MAX30101, Maxim Integrated) for measuring blood oxygenation (SpO₂) and the thermometer (MAX30205, Maxim Integrated). Both ICs are controlled by the BLE SoC through I2C protocol. The power management circuit for the embedded and detachable battery operation includes a voltage regulator that drops the voltage down to supply voltage of various ICs either at 3.3V or 1.8V. The wireless power transfer platform includes the inductive coil tuned at 13.56 MHz, a full-wave rectifier, two-stage voltage regulator, and a heat sink.

FIG. 6B schematically shows an exploded view of a chest EES sensor with the embedded battery modular power supply options according to certain embodiments of disclosure. As shown in FIG. 6B, the chest EES sensor 600 includes a primary cell, which is formed by a plurality of flexible circuits 610 being folded and disposed together with multiple magnets 650 between the top encapsulation 640 and the bottom encapsulation 670. Further, multiple magnets 630 are encapsulated between the top encapsulation 640 and the battery encapsulation 620. The chest EES is mounted on the chest to record electrocardiograms (ECGs), mechano-acoustic signals, and skin temperature.

FIG. 6C schematically shows the formation of the chest EES sensors as shown in FIG. 6B, in which the flexible circuits 610 are formed by disposing the circuit chips on a flexible substrate 612 (which can be a 2-layer printed circuit board), forming a flat structure. The flat structure is then folded, and the folded structure is mounted altogether with the encapsulations to form the Chest EES sensor 600. The other EES, referred as the Limb EES, mounts on the limb such as the base of the foot, toe, and hand to record photoplethysmograms (PPGs) by reflection mode and peripheral skin temperature. FIGS. 6D, 6E and 6F shows examples and photographic images of the flexible and wireless sensors according to certain embodiments of invention. The unique construction of the Chest EES involves foldable islands for optimal distribution of IC components necessary for wireless communication in Bluetooth Low Energy (BLE) protocol, sensing physiological signals, wireless charging circuitry for an embedded battery (Li-polymer, 45 mAh) operation or magnetically releasable power supply circuitry compatible to two different sources: (1) removable battery unit consists of a coin cell (e.g. CR1216) and magnets. and (2) battery-free, inductively induced wireless power transfer platform consists of a RF coil tuned at 13.56 MHz and a power regulating circuitry. Connected through umbilical interconnects, the BLE system-on-a-chip (SoC), located in the middle island, controls both the power circuitry and the sensing island, the smallest island (L x W x H = 1.9 x 1.5 x 0.4 in cm) through another umbilical interconnect that consist of the optical and temperature sensor. Similarly, the Limb EES is designed with the unique embodiments optimized for twisting and bending. The longest island consists of the power circuitry supporting the wireless charging of the embedded battery operation or magnetically releasable modular power operation. Such distribution of core units with umbilical interconnects provides flexible wrapping at the multiple limb interfaces: ankle-to-base of the foot for neonates in NICU (optical sensor on the base of the foot), whereas foot-to-toe and wrist-to-hand for older age group in PICU (optical sensor on the toe or hand). Coating with a silicone material (Silbione RTV 4420, Elkem) encapsulates the EES and modular units. The modular power solution has several advantages: (1) prolonged operation lifetime above the point limited by battery capacity that can prevent frequent removal of sensor that adheres to the skin through a hydrogel adhesive, which is often the major factor damaging underdeveloped skin, especially for premature babies with excessively low gestational ages, (2) compatibility to autoclave sterilization of sensors that is otherwise not achievable with an embedded battery, and (3) providing thin sensor profile that allows safe skin-to-skin interaction between the parents and their child. Using the coil associated to an antenna placed under the mattress permits continuous vital signs monitoring of a neonate on the bed with a thin profile platform. Replacing the coil modular unit by a battery unit offers an efficient solution even for events implying physical distance to the bed, such as feeding or Kangaroo Mother Care events. Interchangeable battery units include various lifetimes associated to capacity and size variations. Thinnest profile is achieved using a CR1216 coin cell battery, resulting in a battery module with a maximum thickness of 3mm. The finite element analysis (FEA) of the serpentine interconnects that connects the islands of the Chest EES, which is designed to form vertically buckled interface when compressed, shows that this buckled interface contributes to the flexibility of sensors and reversible, elastic bi-axial strain up to ~503%, thereby providing conformable mounting on the chest of babies, even with extreme curvatures such as pneumothorax. FIG. 6G shows the stretching and bending characteristics of the serpentine interconnects of the Limb EES that is optimized up to the bending radius of 3.9 mm. The FEA result shows the strain characteristics of the overall Limb EES, assumed to be wrapped at the wrist-to-base of the foot interface.

As shown in FIGS. 6D-6F, the flexible nature of the Chest EES allows to have the mounting even on such highly curved surface around the chest due to defeated chest wall, which results in versatility of the Limb EES to be mounted on various skin interface. The Limb EES can be wrapped around ankle-to-base of the foot for babies having small foot size, usually found in NICU. The EES can instead be mounted around foot-to-toe or wrist-to-hand for babies typically older than several months of chronological age. The mechanics of the Limb EES, optimized for twist and bending, makes it suitable to be applied on various age groups.

### Real-time Measurement of Clinical Data in the Neonatal/Pediatric Intensive Care Unit

Continuous wireless data transmission to a computer system that supports real-time data analytics yields results that can be graphically displayed in an intuitive manner for nurses, doctors and parents. Wireless and real-time streaming through BLE mode of operation allows to provide a patient-centric and accurate measurement of vital signs. The Chest EES measures ECGs, the chest movement through the accelerometer, and skin temperature each sampled at 504, 100, and 5 Hz, respectively. The Limb EES measure PPGs and skin temperature sampled at 100 and 5 Hz, respectively. FIG. 7A represents the waveforms of ECGs, PPGs, and the chest movement measured on a neonate real-time streamed to the base station (Surface Pro). A program developed in Python receives data and run real-time signal processing to yield vital signs. A streamlined Pan-Tompkins algorithm composed of filtering and R-peak detection process yields HR. The chest movement data measured from Z-axis is processed through band-pass filtering (*f_{c1}* = 0.1 Hz, *f_{c2} =* 1 Hz) and a streamlined automatic multiscale-based peak detection (AMPD) to yield RR.

FIG. 7B shows one-hour long representations of HR, SpO₂, RR, and temperature data obtain by EES system on a neonate. These representative data agree well with those captured simultaneously using clinical gold standard instruments (Intellivue MX800, Philips for HR and SpO₂; Giraffe Omnibed Incubator, GE for temperature; direct physician observation of respiratory rate) with outputs derived from a software license (BedMaster, Anandic Medical Systems). Calculated vital signs show no measurable difference compared to same vital signs from gold standard (Intellivue MX800, Philips). The inventors elected to use direct physician observation of respiratory rate given the known inaccuracies in deriving respiratory rate in critically ill newborns and children from ECG, PPG, or airflow measurements in non-intubated subjects.

Calculation of SpO₂ involves with algorithms known for an effective motion artifact reduction, which is critical to calculate accurate value as babies in NICU and PICU are often fidgeting (FIG. 7C). Band pass filtered (f*_{c1}* = 0.5 Hz, f*_{c2}* = 5 Hz) and normalized PPG signals is processed further with the continuous wavelet transform (CWT) that constructs continuous time-frequency analysis of the signal. CWT is effective in detecting rapid changes in frequency in the time domain that can be caused by motion-driven artifact, which serves as the first motion artifact reduction stage. Following with the power ratio calculation and getting the median value, the signal processing further suppresses motion artifact by Discrete Saturation Transform (DST) algorithm. DST algorithm involves with an adaptive filtering and determination of family of the reference noise signal and true signal based on optical density ratio. The adaptive filtering automatically cancels noise content, which is based off the pre-determined reference. This series of signal processing is occurring in real-time to yield an accurate SpO₂ value.

Quantitative comparison in FIG. 7D using the Bland-Altman method further supports good agreement between EES and the gold standard. As shown in FIG. 7D, the mean difference for HR, SpO₂, RR, and temperature is -0.11 beat per minute, 0.18 %, 0.45 breath per minute, and 0.2 °C, respectively. The standard deviation for HR, SpO₂, RR, and temperature is 1.56 beat per minute, 2.9 %, 1.64 breath per minute, and 0.26 °C, respectively.

### Kangaroo Mother Care and Cry Analysis

Beyond to the ability to measure core vital signs as accurate as existing gold standard, EES sensors provide more advanced functionality. FIG. 8A shows Kangaroo mother care (KMC) tracking and vital sign monitoring, and FIG. 8B shows cry signal analysis of neonatal patients according to certain embodiments of the disclosure.

Specifically, panel (a) of FIG. 8A represents the posture detection using the motion information from the accelerometer of the Chest EES. Numerous literature reports that KMC can promote the health condition of a baby and it is widely happening especially in low-resources settings as a secondary clinical practice. Identifying KMC activities out of normal motion-related outcome is therefore important to characterize the effect of KMC. According to the guidelines from the World Health Organization (WHO), neonates are held in an upright position on the parent's chest during KMC, with the neonate's abdomen placed at the level of the parent's epigastrium, and the neonate's head turned to one side to allow eye contact with the parent. The neonatal body position during KMC is distinctly different from the neonatal body position during typical daily activities. KMC position demonstrated an acceleration force of -0.048 ± 0.003 g, - 0.786 ± 0.003 g, and 0.637 ± 0.003 g in the x-, y-, and z-direction respectively, corresponding to an angle of 90.418° ± 0.156°, 138.178° ± 0.249°, and 47.360° ± 0.230° respectively with the gravity vector. Neonatal supine, typical neonatal hold (where neonate is in horizontal position instead of upright), and right lateral position showed significant difference when compared with the KMC position (p = 0 for all positions compared with KMC) in 3-axes acceleration force. These results demonstrate the feasibility of identifying KMC events from other daily activities using this device. Panel (b) of FIG. 8A provides the three-dimensional representation of the posture information obtained in NICU during a KMC session. KMC events demonstrated an angle of 118.5 ± 43.4 °, 103.7 ± 5.3 °, and 52.0 ± 22.3 ° in the x-, y-, and z- direction respectively, with gravity vector as the reference. Verification in the neonatal resting event (right lateral position), typical neonatal holding event, and KMC event in the clinical environment showed significant differences in 3-axes acceleration force (p = 0, Fig. 4B, N = 3). Panel (c) of FIG. 8A demonstrates the successful monitoring of neonatal skin temperature at the posterior and periphery throughout the duration of the study. The neonatal patient in panel (c) of FIG. 8A showed peripheral temperatures of 32.84 ± 0.25 °C, 37.59 ± 0.03 °C, 34.98 ± 0.16 °C respectively during neonatal resting at right lateral position, KMC, followed by a post-KMC neonatal resting event. This demonstrates the ability of KMC to serve as a cost-effective alternative to incubator care, providing effective thermal control and protection for the neonate, as shown in previous studies. The posterior temperatures of 36.38 ± 0.09 °C, 36.27± 0.26 °C, and 36.60 ± 0.14 °C during the same positions respectively did not show clinically-relevant differences that represents the ability of EES to not only track KMC sessions, but also monitor vital signs during KMC activities to provide feedback for the parents and caregivers on the physiological state of the neonate.

In the developmental period of the neonatal neurological system, early diagnosis of neurological disorders enables intervention and treatment in a timely manner. Cry analysis has been reported as a non-invasive method to analyze the neurophysiological state of the neonate, such as birth trauma, brain injury or pain stress. Capturing crying signal has typically involved audio measurements, where signals may easily be contaminated with non-specific audio signals in the environment. The inventors utilize the accelerometer functionality of the Chest EES to capture the mechanical vibration from the neonatal skin during cry activities. FIG. 8B shows the time-frequency signal captured from the neonatal chest for the capturing of cry events and measurement of cry durations. Crying signal had distinctive frequencies from other physiological signals such as heart beat (1 - 3 Hz) or muscle tremor (< 20 Hz). Panel (a) of FIG. 8B shows the spectrogram of a typical cry signal compared with resting events or patting on the neonate. Crying activity reflected a strong signal between 400 - 500 Hz, which was distinctive from patting signals where strong harmonics of patting-induced muscle tremor induced a periodic pattern in the frequency power analysis (see also the statistics of crying detection in FIG. 8C). Panel (b) of FIG. 8B shows the frequency power spectrum upon fast Fourier transform processing of a crying event at a 0.2-s time frame, where a local maximum at 460 Hz was observed. The fundamental frequency of crying signals obtained from NICU patients was 410.7 ± 47.9 (Fig. S8, N =3), which was in accordance with results from previously-reported cry studies. Panel (c) of FIG. 8B shows the duration of cry per neonate identified by the Chest EES. A total of 11 cry events were recorded, which showed no difference when compared with manual recording at the clinical bedside (N = 3). In addition, the duration of each cry event identified by Chest EES was compared with clinical recording, showing an average difference of -3.9 ± 13.9 seconds (indicating an average difference of 4.5%), demonstrating high accuracy in crying duration analysis.

FIGS. 8A and 8B show a proof-of-concept of KMC event tracking and neonatal vital sign monitoring in NICU. KMC is especially important in low-resource countries, where medical facilities are limited. It provides a low-cost alternative to incubator care, enhancing vital sign stability, decreasing risk of infection, and lowering neonatal mortality and morbidity. The KMC identification feature of the Chest EES (FIG. 8A) enables parents and physicians to keep track of therapeutic skin-to-skin care activities. In addition, this platform enables wireless, real-time capturing of vital signs during KMC sessions, enhancing bonding between the neonate and the parent while monitoring the physiological status of the neonate. The inventors envision the Chest EES to provide KMC tracking and wireless vital sign monitoring for the enhancement of neonatal care, both in NICU and out-patient environments, including low-resource countries. The study shown here was a 3-hour study where neonates where in pre-determined positions (right lateral-KMC-right lateral).

Neonatal cry is one of the main communication methods for neonates to express distress. The analysis of cry activities and patterns have recently been suggested to reflect the neurodevelopment and physiological states of neonates, including the detection of the Sudden Infant Death Syndrome, asphyxia, congenital heart diseases, and Respiratory Distress Syndrome. The inventors have demonstrated the ability of the Chest EES to capture neonatal cry signals in NICU based on the distinct fundamental frequency of cry activities (FIG. 8B). The successful capturing of cry events and high-level correlation of cry duration by the Chest EES provides evidence of the successful development of a cry detection platform. It should be noted that the clinical log used to compare such crying activities were taken by hand, introducing human error into the comparison, indicating that the precision of the device should be further validated with alternative methods of greater temporal resolution. In addition, more in-depth analyses of cry patterns and vocal features may enhance the precision and functionality (i.e. detection of potential health risks) of this platform, additional parameters of interest include amplitude of cry, timing variables (onset, duration, inter-utterance interval, etc.), and the change in fundamental frequency with respect to time. Further analysis on the cry patterns of both healthy and pathological neonates, coupled with the real-time, multi-modal vital sign information, will enable the Chest EES to provide further insights into neonatal health management.

### Time-synchronized Bi-nodal Communication for Non-invasive Blood Pressure Monitoring

Blood pressure reflects hemodynamics states and cardiovascular health whose disorders are common in neonates and children admitted to the neonatal and pediatric intensive care unit, and thus counts among essential vital signs to monitor. Measurement in current clinical practice involves with invasive catheter to the arterial line, which creates significant barrier to both parents and caregivers. In this example, the inventors present the non-invasive method of calculating blood pressure by the pulse arrival time (PAT) that has been highlighted as a promising surrogate for blood pressure by numerous literatures. PAT is defined as the time required for a pulse pressure wave to travel from the heart to a distal extremity and depends on vascular system geometry and elasticity as well as on blood pressure. Time-synchronization between two physically distant EES sensors is the key to achieve accurate PAT readings. It is achieved with the multi-protocol capability of the BLE SoC, allowed by the timeslot API. Every one second while each EES communicates with the base station (Surface Pro) in BLE mode of operation independently, the Limb EES transmits its local clock information to the Chest EES synchronizes time difference between local clocks of two EES (FIG. 8D). The result is achieving time delay of less than 1ms with the average standard deviation of 3.6 ms over the continuous running of 24 hrs of operation (FIG. 8D), which allows the EES to calculate accurate PAT readings between ECG R-peak and the foot of PPG signal. The two measures of the validation between PAT-derived SBP matches with the gold standard (BP742N 5 Series, Omron) measured on a healthy adult during two cycles of rest and exercise show no comparable difference in trend. FIG. 8E presents the PAT-derived SBP measured on two different infants in the PICU.

The data shown in FIG. 8E confirm capturing PAT with the platform is a promising method to continuously monitor blood pressure trends for patients in the neonatal and pediatric intensive care units through a continuous and non-invasive probing technique, reducing risks and improving comfort associated to the measurements. Conventional blood pressure measurements are indeed either non-invasive but non-continuous, relying on inflation of a cuff that applies pressure to the arm to stop the blood flow, which cannot be repeated at short intervals, or continuous but invasive as based on direct pressure measurement through an intra-arterial cannula that provides gold standard readings, but increases risks of bleeding, hematoma, nerve injury and infections. The capabilities of the soft wireless binodal platform in terms of PAT measurements offer a soft wearable alternative to continuously measure blood pressure trends in fragile populations. To date, relationship between blood pressure and PAT has been mainly studied in adult cohorts and in infant cohorts in the context of sleep studies, generally with wired devices. Exploration of this surrogate of blood pressure in neonatal intensive care unit is limited and recent and the use of a wearable platform instead of wired devices is particularly adapted to that population.

### Methods

### Fabrication

Fabrication includes soldering electronics components onto a flexible electronics board obtained through laser process. Embedding the assembled circuit board into a soft silicone elastomer shell then avoids any unwanted exposure of electronics parts. For embedded battery version of the device, a Silbione RTV 4420 (Part A & Part B, mixed with 5% of Silc-Pig silicone opaque dye) layer casted in an aluminium mold provides a top shell. A Silbione 4420RTV bottom layer spin coated at 250rpm results in a flat bottom layer. Both layers are fully cured in a 100°C oven for 20 minutes. For ECG device encapsulation, a layer of 3M 96042 double-coated tape laminated onto the flat bottom Silbione RTV 4420 layer allows for good contact of the electronics part with bottom side. For PPG device encapsulation, the flat Silbione RTV 4420 bottom layer stays bare. Using a CO₂ Universal laser cutter, openings cut on the bottom layer allow electrical contact of ECG electrodes as well as optical transparency for the LED module of the PPG. For ECG device, the flexible circuit board adheres to the 3M 96042 double-coated tape layer, and Silbione RT GEL 4717 added at left, middle and right part of the device results in a soft cushioning for the folded electronics board parts. Top and bottom layers finally assembled using uncured Silbione RTV 4420 are placed in a 100°C oven for 50 minutes, resulting in a sealed encapsulation of the device. For PPG device, a thin layer of transparent Silbione RTV 4420 is spin-coated at 250rpm on the bottom side and cured for 20min in a 100°C oven to provide a complete seal of the LED module. Laser-cutting finally provides a clean cut for the outline of both devices.

Modification of the encapsulation process for the sensor part of modular device include the replacement of the top shell by a flat Silbione RTV 4420 coated with 3M 96042 double-coated tape with laser-cut holes to allow exposure of magnets soldered onto the board. In addition, thin profile battery (coin cell and Li-Polymer) or coil encapsulated separately benefit from drop casting technique to achieve thin profile of encapsulation together with soft tapered edges.

### Synthesis of carbon black PDMS (CB-PDMS)

To a glass slide coated with Ease Release 200 (Mann Release Technologies) was applied tape masks to generate CB-PDMS 250 µm thick films. To a 200 mL round-bottom flask with a stir bar was weighed out 4.5 g of carbon black and 15.0 g of Sylgard 184 base. Both components were dissolved in n-hexanes (~100 mL) and stirred vigorously for 10 min at room temperature. To the mixing solution was added 1.5 g Sylgard 184 curing agent diluted 10-fold with hexanes, and the reaction was stirred for 2-3 min. Solvent was rapidly removed and polymer simultaneously degassed with via rotary evaporation at 40 °C until a smooth spreadable paste was achieved. Polymer was spread onto glass molds, ensuring no cracks from excess n-hexanes evaporation with a flat edge. Samples were cured overnight at 70 °C to generate CB-PMDS films.

### CB-PDMS Encapsulated Devices

The top shell was prepared as described above. Briefly, a CB-PDMS sealed bottom layer was prepared by spin coating Silbione 4420RTV and curing as described above. A CO2 Universal laser cutter was used to generate sensor openings with the same dimensions. CB-PDMS electrode pads were cut in the same shape with an excess overlap of 2 mm on all edges. Both the Silbione bottom layer and CB-PDMS pads were corona treated with a BD-20A High Frequency Generator (Electro-Technic Products, Inc.) for 40 sec, and pressed together for 15 sec and cured overnight at 70 °C. To the cured bottom layer was laminated a layer of 3M 96042 double-coated tape that was cut into the shape of the device with holes for the pads. Double-sided 3M electrical tape adhesive was used to adhere the CB-PDMS to the Au-electrodes. The device components and seal between top and bottom layers was carried out as described above.

### Waterproofing Analysis of CB-PDMS Sealed Devices

Non-functional devices CB-PDMS sealed devices, were prepared by replacing the electronic components with Drierite to monitor water permeability. Nonfunctional devices were submerged at 37 °C in 1x DPBS and weight changes were measured. A functional CB-PDMS sealed device, internally lined with three moisture indicators, was incubated continuously at 70 °C in 1x DPBS. Daily measurements of ECG devices were measured until device failure.

### PAT time sync characterization

The inventors characterized time synchronization between the two nodes (ECG and PPG) through a bench top validation experiment: a two-channels function generator provided periodic signals (20ms 3.5V square pulses separated by 1s) with a controlled time delay between the two channels. By connecting first channel to appropriate ECG layout pins and second channel to a red LED blinking on top of the PPG optical module, the inventors successfully demonstrated that the time delay measured through an oscilloscope connected to the function generator matches the time delay measured by the binodal system with the mean delay less than 1ms with the average standard deviation of 3.6 ms (FIG. 8D).

### Autoclavability testing

Autoclavability test of sensor modular part (including no battery) and magnets have been performed using a Heidolph Tuttnauer 3545E Autoclave Sterilizer Electronic Model AE-K. Sterilization included a temperature ramp to 121°C and a subsequent 15 minutes sterilization time, followed by drying. This process resulted in no alteration of devices performances, successfully demonstrating feasibility of autoclave sterilization of the platform.

### Temperature Sensor Characterization

The accuracy of temperature sensor was determined using reference thermometer (Fisherbrand^{™} 13202376, Fisher Scientific ) measurements as standard. The thermometer of EES and the reference thermometer were both placed in a hot water bath that was heated to 42 °C and cooled to room temperature. During the cooling period, the temperature measurements between EES and the thermometer were recorded to characterize the precision of the temperature sensor in EES on the temperature range of 30 °C to 41°C.

### Data Analysis and Algorithms - KMC and Cry Analysis.

KMC analysis was based on accelerometer measurements with a sampling rate of 100 Hz. The accelerometer was calibrated by aligning the x-, y-, and z-axes with the gravity vector and correlating the accelerometer signals with gravity force. The acceleration signal was processed by a Butterworth low pass filter of a cutoff frequency at 0.1 Hz and the angle of the device axes to the gravitation force was calculated through trigonometry processing. Accelerometer signals of the x-, y-, and z-axes were 3-dimensionally plotted and correlated with clinically recorded body positions.

Cry signal recording was achieved by EES with a sampling rate of 1600 Hz. The accelerometer signal was processed by a Butterworth high pass filter, 20 Hz cutoff frequency. Fast Fourier transform was performed on 200 ms segments. Cry event was identified where local maxima between 350 Hz and 500 Hz were significant, and periodic harmonics from lower frequency signals (such as patting) were excluded.

### EXAMPLE 2

This example, related to one aspect of the disclosure, relates to an application of skin-interfaced biosensors and pilot studies for advanced wireless physiological monitoring in neonatal and pediatric intensive care units.

Pilot studies on patients in NICU and PICU demonstrate the feasibility of a pair of soft, skin-interfaced wireless devices to capture HR, SpO₂, RR, as well as core and peripheral temperature with high levels of reliability and accuracy as compared with clinical standard monitoring systems that use conventional, hard-wired interfaces. In fact, the data indicate that in many cases the wireless operation and the gentle, mechanically stable measurement interfaces reduce the magnitude and prevalence of noise artifacts associated with motions and other parasitic effects, compared to wired systems. In addition to these basic vital signs, time synchronization techniques yield data that serve as promising surrogates of SBP, thereby offering the potential to bypass the use of cuffs for episodic measurements and arterial lines for continuous tracking⁴⁸. Predicate results on adults and pediatric populations lend confidence in the findings presented here, as the first measurements that exploit accelerometer-based PTT on patients in the NICU and PICU. The device designs and the simplicity of the user interface suggest opportunities for deployment outside of traditional NICU/PICU facilities, into the developing world and even into the home. The availability of continuous, high quality digital data streams in these and other contexts suggest opportunities for use of advanced analytics to extend the range of utility in clinical and remote care.

Another important outcome of the work is in demonstrated capabilities for capturing advanced and unusual physiological signals such as SCG, body orientation, activity and vocal biomarkers. Cardiac monitoring with SCG yields important data to complement those associated with ECG, with enhanced utility in early detection of cardiac complications. Although SCG measurements are reported on adult populations, their use in routine clinical practice is rare and is absent in neonatal and/or pediatric contexts due, at least partly, to the high degrees of curvature of the chest and the fragility of the skin surface. The same data streams yield, through digital filtering techniques, information on body orientation and activity, which are relevant to identifying and quantifying KMC, feeding, holding, resting, patting, and potentially sleep patterns. Quantifying such measures has potential to provide insight into the role these activities have on physiologic stability, neurodevelopmental, and other short and long term outcomes. The collective suite of measurements may allow optimization and enhancements in care, in which vital signs and other parameters can serve as guiding signatures of efficacy. Here, as well as in traditional vital signs monitoring, advanced analytics, including methods such as machine learning, may be very powerful. Such techniques could offer particular value in the analysis of neonatal cry, as a rich source of information that represents the main method for neonates to communicate distress⁵⁵. Studies in controlled settings using microphone recordings indicate that cry patterns reflect neurodevelopment and physiological status, with potential relevance to the detection of sudden infant death syndrome, asphyxia, congenital heart diseases, and respiratory distress syndrome⁵⁷. The platforms introduced here eliminate difficulties associated with ambient sounds in the noisy environments of the NICU and PICU, thereby creating an opportunity to exploit this relatively underexplored, yet rich source of information in settings of practical interest.

The robustness of the platforms, the options in power supply, the sealed/waterproof construction, the soft mechanics, the skin-safe adhesive interfaces with no instances of skin tearing or dermatitis associated with the devices, the compatibility with established sterilization techniques, the re-usability of the devices, and the alignment of the constituent components, materials and designs with advanced manufacturing practice suggest broad deployability. The outcomes have the potential to enhance the quality and breadth of information for physicians, nurses and parents responsible for the care of neonatal/pediatric patients. A growing base of multilateral physiological data, most notably continuous heart activity, respiration, temperature, blood pressure, motion, body orientation, and vocal biomarkers, coupled with advanced learning algorithms, may facilitate early diagnosis of many common complications in these populations, including seizures, and apnea, upon extensive collection and analysis of data from relevant clinical studies. The core technology, beyond neonatal and pediatric critical care, has clear applications in post-acute monitoring, outpatient or home settings, trauma situations, and low-resource environments.

In certain embodiments, a soft, skin-like electronic system is provided to address these unmet clinical needs. Evaluation studies in the NICU confirm capabilities for clinically accurate measurements of heart rate (HR), blood oxygenation (SpO₂), temperature, respiration rate (RR) and pulse wave velocity (PWV) in the NICU. However, this system is limited by (1) the modest maximum operating distances (~30 cm) supported by NFC protocols used for power transfer and data communication, (2) the mechanically fragile nature of the ultrathin, compliant mechanics designs, (3) the sufficient, but constrained range of measurement capabilities, and (4) the demand for highly advanced device configurations, capable of fabrication only in specialized facilities with customized tools. The results reported below adopt and extend similar principles in soft electronics design, but in mechanically robust, manufacturable systems that rely on Bluetooth technology to circumvent these limitations. These systems include a range of options in operation and power supply that address a broad spectrum of clinical use cases and provider preferences, ranging from modular primary batteries to integrated secondary batteries to wireless power harvesting schemes. These platforms additionally support important modalities in monitoring that lie beyond both the standard of care and the capabilities of the previously reported systems. These include the ability to: (1) monitor movements and changes in body orientation, (2) track and assess the therapeutic effects of KMC and other forms of hands-on care, (3) capture acoustic signatures associated with cardiac activity by capturing mechanical vibrations generated through the skin on the chest wall reflective of valvular function (4), record vocal biomarkers associated with tonality and temporal characteristics of crying, and (5) quantify pulse wave dynamics through multiple measurements, as a reliable surrogate for systolic blood pressure.

The ability for this system to provide addition quantitative information on hemodynamic and cardiovascular health states beyond the core vital signs of heart rate, respiratory rate and blood oximetry holds direct relevance to the management of patients in the NICU/PICU. Visualization of heart vibrations, referred to as a seismocardiogram (SCG), is rarely performed in general clinical practice, especially in the NICU/PICU, yet it provides essential information on the mechanical outcomes of myocardial activity, valve motions and other features that are absent from ECG data. Episodic measurements of BP in current clinical practice on neonates and pediatric patients involve miniaturized, but otherwise conventional, BP cuffs that wrap around the limbs, while continuous tracking requires catheter-based pressure sensors (arterial lines) that insert into peripheral arteries. Both procedures, particularly the latter, are invasive and involve multiple risk factors, to an extent that they are used only in limited cases despite the essential utility of the information. The capabilities of the system enable the ability to address aspects of neurological, respiratory, and pathological disorders that are common in premature neonates and can lead to abnormalities in vocalization, range of motion, and posture control. Quantitative, continuous tracking of such behavior offers the potential for early detection of complications associated with birth trauma, brain injury or pain stress. Measurements of movement and physical activity specifically can provide insights into sensorimotor development. These same data can also inform effective methods for neonatal care such as KMC, a therapeutic skin-to-skin "treatment", in which a pediatric patient is held against a parent's chest in a manner that lowers mortality, stabilizes heart rate, temperature, and respiration rate, and decreases the risks for infection.

The device and system design of this example is similar to those as used in Example 1. The technology platforms, measurement capabilities, clinical effectiveness, and safety through pilot studies on the same 50 patients in FIG. 5 across a wide range of ages in both the NICU and the PICU are described hereinafter in details. Among the 50 patient, a change in skin score was determined using modified Neonatal Skin Condition Scale (3-9). The scale is used to score the underlying skin 15 minutes after removal of each sensor. The score is compared to the pre-testing skin. Higher scores indicate greater skin erythema (1-3), dryness (1-3), and breakdown (1-3). A perfect score is 3 where there is no evidence of skin dryness, erythema or breakdown. A score of 9 is the worst indicating very dry skin with cracking/fissures, visible erythema in > 50% of skin underneath the sensors, and extensive breakdown. The average change in the score (negative change suggests improvement) was -0.02. Only 2 subjects (4%) of subjects exhibited an increase in the scale, which was limited to a 1-point increase.

### Results

### Device and System Design

This example uses a modular battery unit for power supply in a design that allows for gentle placement on the curved skin of the chest (chest unit) via a thin hydrogel coupling layer to record electrocardiograms (ECGs), acoustic signals of vocalization and cardiac/respiratory activity, body orientation and movements, and skin temperature, all enabled by a BLE SoC and associated collection of sensors. The overall layout includes a thin, flexible printed circuit board (PCB) and mounted components, configured in an open design with serpentine interconnect traces. The construction involves folding of distinct, but connected, platforms as a key step in assembly and packaging. Quantitative insights from three-dimensional finite element analysis (FEA) of the system-level mechanics helped to define an optimal distribution of the active components to reduce the lateral dimensions of the device by ~250 %. A pre-compression process in the assembly forms buckled layouts in a serpentine configuration to enhance flexibility and stretchability. An elastomeric enclosure with an inner silicone gel liner (~300 µm thick, ~4 kPa) enhances the device softness ensuring compatibility with the fragile skin and highly curved anatomical features of neonates born at the lowest gestational ages. A pair of thin, conductive elements formed using a doped silicone material (carbon black in polydimethylsiloxane, abbreviated as 'CB PDMS'; bulk resistivity of 4.2 Ω.cm) serve as soft electrical connections to corresponding gold electrodes on the flexible printed circuit board and to conductive hydrogel skin interfaces for ECG measurements. The result is a soft and completely sealed, waterproof system with applicability across a wide range of settings, focused on but not limited to the NICU and PICU.

### Novel Power Management Schemes

The modular battery unit couples to the device mechanically and electrically through pairs of matching sets of embedded magnets, thereby: (1) allowing replacement of the battery without removing the device from the patient with the aim to minimize disruptions in clinical care, decrease the burden on clinical staff, and consequently reduce risks of skin injury; (2) enabling removal of the battery to allow autoclave sterilization of device; and (3) mechanically decoupling the battery from the device to improve the bendability and, therefore, the compliance at the skin interface. The magnetic scheme also allows for other options for power supply, not only in choices of battery sizes, shapes and storage capacities (and therefore operational lifetimes), but also in alternative modalities, including battery-free schemes that rely on wireless power transfer. As an example of this latter possibility, a magnetically coupled harvesting unit can be configured to receive power from a transmission antenna placed under the bed and designed to operate at a radio frequency of 13.56 MHz with a negligible absorption in biological tissue.

Modular batteries are encapsulated with various shapes, showing the possible compatibility with choking hazard prevention requirements. Given that a removable battery can act as a swallowing and choking hazard in older infants, the battery can be designed with geometries that are larger than the minimum size requirements for consumer products used by children under the age of three (see FIG. A). A third option is to provide a wirelessly rechargeable battery (Li-polymer, 45 mAh) which lies within the sealed enclosure of the device to eliminate any external connections.

### Sensor Mechanics and Design

FIG. 9B shows schematic illustration of the serpentine interconnects used in a chest unit, and FIG. 9C shows computational demonstration of the mechanical properties of a chest unit according to certain embodiments of the disclosure. The three sub-systems are linked mechanically and electrically by soft serpentine interconnects that provide high stretchability and conformably comply with physiological deformations when the device is mounted in the human body. The soft serpentine interconnects consist of two 12µm-thick copper layers encapsulated in polyimide (PI) and separated by 25 µm in the out-of-plane direction. Each copper layer features three serpentine traces with a width W = 75 µm and the in-plane separation between the traces is 75 µm. The total thickness of the serpentine interconnects is 99 µm. In the chest unit, the serpentine interconnects encapsulated with polyimide (PI), the folded configuration, and the soft enclosure with gel liner lead to a uniaxial elastic stretchability that exceeds ~33 % at the device level, corresponding to a ~500 % stretchability in the interconnects prior to encapsulation in the outer silicone shell (FIGS. 9B and 9C). The gel (~300 µm thick, ~4 kPa modulus) provides strain isolation between the folded islands to reduce the stresses at the skin interface to levels below the thresholds for sensory perception (20 kPa) for uniaxial stretching of up to 20%, a value at the high end of the range expected in practical use. The resulting elastic bending radius and equivalent bending stiffness are ~20 mm and ~9.6 Nmm², respectively, as shown in panel (c) of FIG. 9C. These mechanical characteristics ensure soft, irritation-free skin interfaces, even in cases of extreme curvatures encountered with small babies and/or low gestational ages.

This limb unit features a layout that facilitates wrapping around the foot, palm or toe-this accommodates neonates and pediatric patients of varying ages and anatomies. The overall design of the limb unit is with umbilical interconnects that can bend to radii as small as ~3.9 mm twist through angles as large as 180° and elastically stretch to uniaxial strains as high as 17% (see FIGS. 9D and 9E). FIG. 9D schematically shows a representative interconnects used in the limb unit according to certain embodiments of the disclosure. The soft serpentine interconnects consist of two 12 µm-thick copper layers encapsulated in polyimide (PI) and separated by 25 µm in the out-of-plane direction. Each copper layer features three serpentine traces with a width W = 75 µm and the in-plane separation between the traces is 75 µm. The total thickness of the serpentine interconnects is 99 µm. FIG. 9E schematically shows mechanical characteristics of a limb unit according to certain embodiments of the disclosure, where the strain distribution in the encapsulation layer (left) and copper layer (right) of a representative interconnect during panels (a) stretching, (b) twisting, (c) bending at the radius of 3.9 mm, and (d) the overall bending mechanics in a limb unit. The fundamental design features of the limb unit are similar to those of the chest unit, but in configurations that anatomically match different limb interfaces: ankle-to-sole of the foot for neonates in NICU and wrist-to-hand and foot-to-toe for larger, pediatric patients in the PICU.

The chest unit includes a wide-bandwidth 3-axial accelerometer (BMI160, Bosch Sensortec), a clinical-grade temperature sensor (MAX30205, Maxim Integrated), and an ECG system that consists of two gold-plated electrodes. The limb unit includes an integrated pulse oximetry module (MAX30101, Maxim Integrated) for measuring dual wavelength PPGs and a temperature sensor (MAX30205, Maxim Integrated). The power management circuit for battery operation uses a voltage regulator to provide supply voltages required for the various components (3.3V or 1.8V). The modular battery-free platform includes an inductive coil tuned to 13.56 MHz, a full-wave rectifier, and a two-stage cascaded voltage regulating unit.

### Clinical Studies on Neonatal/Pediatric Patients in the Intensive Care Unit

The soft mechanical properties and the wireless modes of operation are critically important to effective use on neonatal and pediatric ICU patients, particularly when located at highly curved regions of anatomy on a limited surface area. Wrapping around the ankle-to-base of the foot is effective for premature neonates, as commonly encountered in the NICU. Other options include mounting around the foot-to-toe or the wrist-to-hand, typically most suitable for babies with chronological ages greater than 12 months. These mounting options enhance nearly all aspects of routine and specialized procedures in clinical care, ranging from intimate contact during KMC and parental holding to feed, change diapers, and bathe the infant.

### Real-time Measurement of Clinical Data in the Neonatal/Pediatric Intensive Care Unit

Continuous wireless data transmission to a computer system that supports real-time data analytics yields results that can be graphically displayed in an intuitive manner for nurses, doctors and parents. The chest unit measures ECGs and skin temperature, together with a rich range of information that can be inferred from data collected with the high-bandwidth, 3-axis accelerometer, including SCGs, respiration rate and others, with sampling frequencies of 504 Hz (ECG), 0.2 Hz (temperature) and 100 Hz (SCG). The SCG provides information not only on HR, but also on the systolic interval, the pre-ejection period (PEP), and left ventricular ejection time. The limb unit measures PPGs at red (660 nm) and infrared (IR, 880 nm) wavelengths, and skin temperature, sampled at 100 and 0.2 Hz, respectively.

The streaming raw data from the devices undergoes real-time signal processing on the mobile tablet allowing for dynamic, adaptive vital signs display with negligible time delays. In many cases, relevant information can be extracted from different, independent data streams. FIG. 10A shows signal processing algorithms for panels (a) heart rate, (b) respiration rate, (c) blood oxygenation and (d) pulse arrival and transit time. For instance, HR can be obtained from ECG (panel (a) of FIG. 10A), PPG, and SCG data separately to yield multiple, redundant estimations. Similarly, RR can be determined, not only from any one of these sources of data, but also from the accelerometry measurements (panel (b) of FIG. 10A). Opportunities for exploiting redundancy provided by the full multimodal data suite represent topics of current investigation.

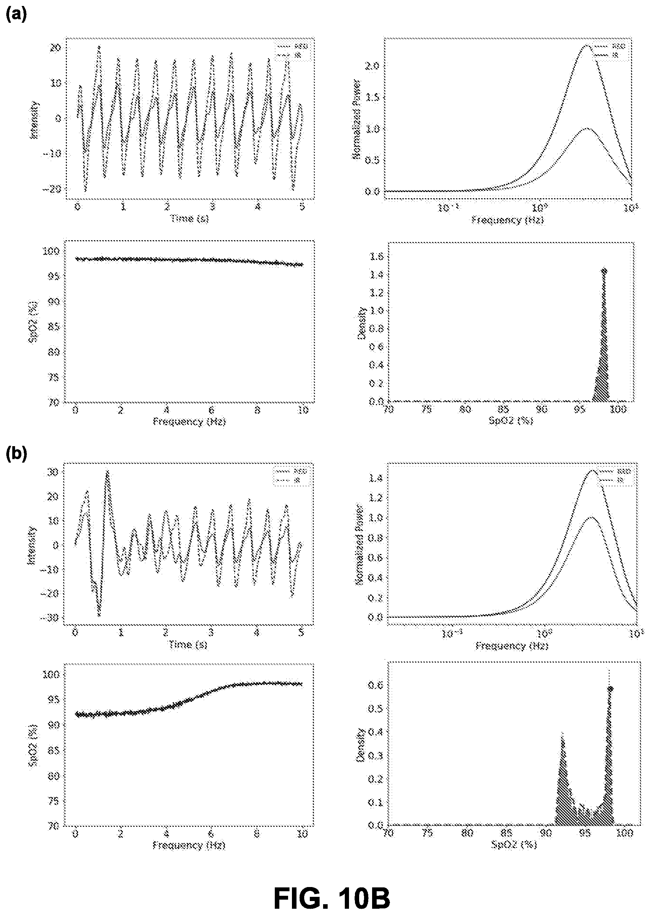

Calculation of peripheral capillary oxygen saturation (SpO₂) exploits dual color PPGs with algorithms designed to minimize the effects of motion artifacts commonly encountered in the NICU and PICU due to naturally occurring movements (panel (c) of FIG. 10A). This platform is effective in detecting rapid temporal changes in frequency, most commonly due to motion artifact, as a simple but effective means to reduce motion artifact effects (FIG. 10B).

The results for HR and SpO₂ are within the regulatory guidelines set by the US Food and Drug Administration (FDA), which require errors less than ± 10 % or ± 5 bpm for HR and less than 3.5 % for Aᵣₘₛ for reflectance mode SpO₂. FDA guidelines for RR monitors under 21 CFR 870.2375 does not specify requirements in terms of accuracy, but a 510(k) cleared bedside monitoring system (Siemens SC 6000) delivers a target accuracy of ± 3 breath per minute. Further safety testing on additional neonates (n = 50) was conducted to evaluate skin tolerability and ensure negligible heat generation from the sensors operating concomitantly with standard monitoring systems. These results included a diverse range of age groups (23 - 40 wks gestational age and 1 week - 4 yrs chronical age), and ethnicities (16 Caucasian, 24 Hispanic/Latino, and 10 Black/African American) (FIG. 5). Thermal safety testing demonstrated no heat generation from either device (FIG. C) in a subset of 3 patients. Finally, no skin adverse events were noted in all 50 subjects as graded by the Neonatal Skin Condition Score (NSCS) (FIG. 5).

### Time-synchronized Bi-nodal Communication for Non-invasive Blood Pressure Monitoring

Pulse arrival time (PAT) and pulse transit time (PTT) are two related but distinct measures with established correlations to systolic BP (SBP). The PAT, calculated from the time difference between the R-peak of ECGs on the chest unit and valley regions of the PPGs on the limb unit, represents the time delay of the pulse pressure wave to travel from the aorta to peripheral limb location at each cardiac cycle. Some studies suggest that the exclusion of the PEP from the PAT may improve correlation with SBP. PTT, calculated from the peak-to-foot time delay between the SCG and PPG waveforms, achieves this exclusion by capturing the residual peak when the aorta valve opens. Ultimately, both PAT and PTT depend on vascular system geometry, elasticity, SBP, and other factors. Extensive studies on adult subjects establish calibrated correlations between PAT, PTT and SBP using both empirical and theoretical models, some of which are clinically approved for monitoring in certain scenarios (e.g. Sotera ViSi Mobile^{®} System). Few studies report the correlation of PAT with SBP in infants, mainly in the context of sleep studies and as screening method rather than a core clinical tool. None report measurements of PTT in this critical care population.

This design integrates synchronous operation of the chest and limb devices, enabling measurements of PAT and PTT for each cardiac cycle. To ensure timing accuracy, once every second the chest unit transmits its 16 MHz local clock information to the limb unit. The result eliminates timing drift to enable a synchronization accuracy of greater than 1 ms, on average, and a standard deviation of 3.6 ms over a continuous, 24 hour period of operation (see FIG. 8D). This scheme requires an additional current consumption of ~0.2 mA compared to the standard mode of operation. The timing interval of one second provides a tradeoff between power consumption and timing accuracy, given that the measured time delays of interest here are typically > 100 ms. The proportional model derives the linear relationship of PAT and PTT data to SBP, shown in the equation 3, in which PT can represent either PAT or PTT $SBP = - a \left(PT\right) + b$ (3)

Calculation of coefficients in the equation involves the linear regression of PAT and PTT data to 5 min of SBP data measured using an A-line, which serves as an initial calibration. The demonstration here of exclusion of PEP in the form of PTT is the first reported in NICU/PICU. Accelerometry data of a chest unit (FIG. 11) shows the correlated behavior of the overshoot of the A-line data with motion artifact. Such modality presents the opportunity to measure the signal quality index to determine the reliability of data at the incidents of movement to derive more reliable SBP output. The results show strong agreement throughout the 5 h measurement period. The mean differences of 1.31 and -1.25 mmHg and the standard deviations of 7.64 and 6.11 mmHg for PAT and PTT-derived SBP values, respectively, indicate their statistical validity. The results are well within the ANSI/AAMI SP10 standard, which requires the mean differences and standard deviations of <5 mmHg and <8 mmHg, respectively. FIG. 12 summarizes the effects for the data.

### Advanced Use Cases: Kangaroo Care and Cry Analysis

In addition to SCG and PTT, several additional important modes of operation follow from further use of data from the high-bandwidth 3-axis accelerometer. Examples include motion/movement (including tracking KMC and infant holding), and measuring vocal biomarkers such as tonality, dynamics and frequency of crying. According to guidelines from the World Health Organization (WHO), KMC involves holding the neonate in an upright position on the parent's chest, with the neonate's abdomen placed at the level of the parent's epigastrium, and the neonate's head turned to one side to allow eye contact with the parent. This body position, which can be precisely and continuously monitored using low pass filtered (0-0.1 Hz) data from the accelerometer of the chest unit, is distinct from those that occur during most other activities and forms of care.

Panel (a) of FIG. 8A presents the device and reference coordinate frames and their relative orientations. Here, phi and theta correspond to rotations around the x- and y-axis, respectively, consistent with the right-hand rule. Panel (b) of FIG. 8A demonstrates measurements of core body orientation using data from a chest unit placed on the back of a neonate. A time dependent reproduction of the orientation results from a straightforward computational approach. Here, a stationary hold in the KMC position yields phi and theta angles of 2 ~ 3 rad and -0.5 ~ 0 rad with respect to the reference frame, respectively. Data collected for the cases of supine, horizontal and right lateral orientations are each significantly different from the KMC position (P-values < 10⁻⁵ for all positions compared with KMC) in terms of rotational angle. Based on the three-dimensional representations and angles obtained in the NICU during a KMC session, the KMC events correspond to 2.85 ± 0.10 rad, -0.29 ± 0.28 rad (data are mean ± std for 2.4 h) in phi and theta respectively. Comparisons between resting (right and left lateral position), holding, feeding, and KMC events in this clinical environment each show significant differences in 3-axes acceleration and rotational angle (P-values < 10⁻⁵, n = 3).

Based on the results of HR, SpO₂, central and peripheral skin temperature, along with a measurement of activity derived from the accelerometry data before, during, and after the KMC study, including removal and return of the neonate to the crib, activity corresponds to the root mean square value of 3-axis accelerometry data after bandpass filtering between 1 and 10 Hz. The data show that skin-to-skin contact during KMC produced a pronounced, gradual increase in the peripheral skin temperature, consistent with expectation and as demonstrated in previous studies. The mean activity levels during rest and KMC events are 0.07 ± 0.02 g/s, while during hands-on care these values are 0.24 ± 0.05 g/s (data are mean ± std for 3 neonates, total 8 hours of KMC/rest and 75 min of hands-on care). These data have potential to provide a quantitative indicator to help minimize the disturbance of neonates during various forms of care, and, therefore, risks of hypopnea, apnea, and oxygen desaturation. Current work seeks to explore this opportunity and to establish methods to use the full set of measurement results to provide feedback on the timing and techniques of KMC, particularly in sessions extending beyond 4 hours, in which the impact on physiological parameters are expected to be enhanced.

In addition to activity, orientation and SCG, the accelerometer also yields information on vocal biomarkers generally, and crying in particular, via analysis of the high frequency components of the data. Cry analysis can serve as a non-invasive method to analyze the neurophysiological state, often influenced by birth trauma, brain injury or pain stress. Crying captured by measurements with microphones are easily confounded by ambient sounds in the environment, a particular challenge in NICU and PICU settings. The accelerometer, by contrast, responds only to mechanical vibratory motions of the chest, and is nearly completely unaffected by ambient noise. Panel (b) of FIG. 8B shows typical data (top) and the time-frequency signal (bottom) captured from a representative neonate. The signals associated with crying have distinctive frequencies (typically between 400 and 500 Hz, with strong harmonics), well separated from other physiological effects such as cardiac activity (1 - 50 Hz) and muscle tremors (< 20 Hz) or from various operations in care such as patting, rubbing or stroking (see panels (a)-(c) of FIG. 13). FIG. 13 summarizes 11 crying events measured in this manner, and in a process of manual recording at the bedside (n = 3 infants). The duration of events captured using these two approaches show an average difference of -3.9 ± 13.9 s (data are mean ± std for 11 cry events) (panel (d) of FIG. 13). The fundamental frequency of 410.7 ± 47.9 (panel (e) of FIG. 13) is consistent with published results.

FIG. 14 shows a global BA plot for heart rate and blood oxygenation obtained in the all population (over 0.4 M data points) according to certain embodiments of the disclosure.

### Online Methods

### Fabrication and assembly of the chest and limb devices

Fabrication involved soldering electronic components onto flexible printed circuit boards patterned using a laser ablation process. Embedding the assembled and folded system into a soft silicone enclosure completed the process. For the chest unit with modular options in power supply, films of a soft silicone material (Silbione RTV 4420; Part A & Part B, mixed with 5% of Silc-Pig silicone opaque dye) formed by spin-cast at 250 rpm and thermally curing (100 °C in an oven for 20 min) on glass slides served as top and a bottom layers for the encapsulation process. Curing of both layers involved heating to 100 °C in an oven for 20 minutes. A cutting process with a CO₂ laser (ULS) defined openings for the ECG electrodes on the bottom layer and for magnets on the top layer. A silicone-based adhesive (3M 96042) bonded the electronics to the bottom layer. Pre-compression of the serpentines during this step ensured high levels of stretchability, with associated enhancements in the bendability. A silicone gel (Ecoflex, Smooth-On) cured at 100°C for 20 min provided a soft, strain-isolating interface layer both below (center part) and above (whole coverage) the electronics. Bonding an overlayer of Silbione finalized the encapsulation process. A drop-casting technique formed coatings of Silbione on top of the various modules for power supply.

Fabrication of the integrated secondary battery version of the device exploited a related encapsulation process, but designed to yield an enclosed air-pocket design as a strain insulation layer to minimize the mechanical load associated with the battery. Here, Silbione cast in a machined aluminum mold served as a top capping layer. A film of this same material, formed as previously described, served as the bottom seal against the perimeter region of the shell to complete the enclosure.

An analogous process defined the encapsulating enclosure for the limb unit, with transparent regions at the location of the LED module for PPG measurements. For all devices, a final laser cutting step yielded a smooth, clean perimeter.

### Preparation of soft, integrated electrodes of PDMS doped with carbon black (CB-PDMS)

The formulation involved the addition of 4.5 g of carbon black to 15.0 g of a silicone prepolymer (Sylgard 184 base) in a 200 mL round-bottom flask containing n-hexanes (100 mL) and stirred vigorously with a stir bar for 10 min at room temperature. Addition of 1.5 g of silicone curing agent (Sylgard 184 curing agent) pre-diluted in 1 mL hexane with continuous stirring for 2-3 min induced polymerization. Rotary evaporation at 40 °C led to simultaneous rapid removal of solvent and degassing of the polymer to yield a smooth paste. Uncured CB-PDMS, spread with a flat edge onto glass slides containing level guides coated with mold release spray (Ease Release 200, Mann Release Technologies), yielded thin solid films of CB-PDMS (250 µm thickness) after curing overnight in an oven at 70 °C. Electrode pads, cut with a CO₂ laser to lateral geometries larger by 2 mm along all edges of the openings for the ECG electrodes on the bottom surfaces of the chest unit, provided overlapping regions for bonding. Treatment of both elastomeric surfaces with a corona gun (BD-20A High Frequency Generator, Electro-Technic Products, Inc.) for 40 s, immediately followed by pressure induced lamination (15 s) and overnight curing at 70 °C in an oven yielded excellent adhesion. A double-sided conductive tape (3M 9719) bonded the CB-PDMS pads to the Au electrodes on the flexible printed circuit board.

### Water Immersion Tests of the Encapsulation Structure

Tests of permeation used platforms with the electronic components replaced with a dessicant (Drierite) (n = 4). Studies involved daily gravimetric measurements following continuous immersion in 1x DPBS (Dulbecco's Phosphate Buffered Saline) at 37 °C. A rapid increase in device weight (>1000 mg in 24 h) at ~19-28 days followed from partial delamination of the perimeter seal between the top and bottom Silbione layers, as opposed to the seal between the CB-PDMS and Silbione. Additional tests with a functional chest unit continuously immersed in 1x DPBS at 70 °C, demonstrated stable operation, evaluated daily, for 18 days.

### Quantifying Time Synchronous Operation

Characterization of time synchronization used a two-channel function generator to provide a pair of periodic signals (20 ms 3.5V square pulses separated by 1s) with a controlled time delay between the two. Connecting one channel to the ECG module and the other to a red LED placed on top of the PPG module, yielded data that validated synchronization to a mean delay of less than 1 ms and a standard deviation of 3.6 ms.

### Testing of Compatibility with Autoclave Sterilization

The tests focused on a chest unit with a modular primary battery and a Heidolph Tuttnauer 3545E Autoclave Sterilizer (Electronic Model AE-K). The process involved a temperature ramp to 121°C, a sterilization time of 15 min, and a drying time of 60 min, performed using a device with the battery removed. Functional tests before and after sterilization revealed no change in performance.

### Characterizing the Temperature Sensor

Measurements of the accuracy of the temperature sensor involved immersion in a water bath, heated to 42 °C and then cooled to room temperature, with simultaneous measurements using a reference thermometer (Fisherbrand^{™} 13202376, Fisher Scientific) as a standard.

### Clinical Testing

The research protocol was approved by the Ann & Robert H. Lurie Children's Hospital of Chicago and Northwestern University's Institutional Review Board (STU00202449) and registered on ClinicalTrials.gov (NCT02865070). After informed consent from at least one parent for all participants, the experimental sensors were placed on the chest and limb (foot or hand) by trained research staff. The sensors were placed in a way as to not disrupt any of the existing gold-standard monitoring equipment. No skin preparation was conducted prior to sensor placement or with sensor removal. The protocol enabled collection times of up to 24 hours. However, medical procedures (e.g. surgery) or imaging required removal of the sensors. Upon removal of the sensors, a board-certified dermatologist evaluated the underlying skin for evidence of irritation, redness, or erosions. Data were transmitted, collected, and stored for further data analysis on a tablet PC (Surface Pro 4, Microsoft) placed out of view from parents and clinical staff. All subjects in the Northwestern Prentice Women's Hospital and Lurie Children's Hospital admitted to the neonatal intensive care unit and pediatric intensive care unit were eligible regardless of gestational age.

### Data Analysis and Algorithms - KMC and Cry Analysis.

KMC analysis relied on accelerometer measurements captured at a sampling rate of 100 Hz. Calibration involved aligning the x-, y-, and z-axes of the device with the gravity vector. Signal processing used a Butterworth low pass filter (3^{rd} order) with a cutoff frequency at 0.1 Hz. Simple trigonometry defined the orientation angle from the acceleration values. Results plotted in three dimensions were correlated to manually recorded body positions. Processing the acceleration signal through a Butterworth bandpass filter (3^{rd} order) between 1-10 Hz, followed by computation of the root-mean-square of the acceleration values along the x-, y-, and z- axes yielded a metric for neonatal activity level, determined each second.

Recording vibratory signatures of vocalization, including crying, involved operation of the accelerometer at a sampling rate of 1600 Hz. Signal processing used a Butterworth high pass filter (3^{rd} order) with a 20 Hz cutoff frequency. Fast Fourier transforms yielded power spectra on time segments with durations of 200 ms. Cry events correspond to spectra with significant peaks between 350 Hz and 500 Hz, with exclusion of harmonics from lower frequency signals (such as those due to patting).

Statistical analysis used a one-way Multivariate Analysis of Variance (MANOVA) via MATLAB, with an assumption that data points for each group are normally distributed. P-value < 0.05 was considered significant.

In certain embodiments, any of the systems and devices described herein may be used to practice any of the methods of the disclosure.

In a further aspect, the disclosure relates to a non-transitory tangible computer-readable medium storing instructions which, when executed by one or more processors, cause the methods as discussed above to be performed.

The foregoing description of the exemplary embodiments of the present invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to activate others skilled in the art to utilize the invention and various embodiments and with various modifications as are suited to the particular use contemplated. Alternative embodiments will become apparent to those skilled in the art to which the present invention pertains without departing from its scope. Accordingly, the scope of the present invention is defined by the appended claims rather than the foregoing description and the exemplary embodiments described therein.

### LIST OF REFERENCES

[1]. Wheeler, D.S., Wong, H.R. & Shanley, T.P. Pediatric critical care medicine : basic science and clinical evidence, (Springer, London, 2007).
[2]. Xu, J., Murphy, S.L., Kochanek, K.D., Bastian, B. & Arias, E. Deaths: final data for 2016. Natl. Vital Stat. Rep. 67, 1-76 (2018).
[3]. Bonner, O., Beardsall, K., Crilly, N. & Lasenby, J. 'There were more wires than him': the potential for wireless patient monitoring in neonatal intensive care. BMJ Innov. 3, 12-18 (2017).
[4]. Bowdle, T.A. Complications of invasive monitoring. Anesthesiol. Clin. N. A. 20, 571-588 (2002).
[5]. Cilley, R. Arterial access in infants and children. in Seminars in pediatric surgery, Vol. 1 174-180 (1992).
[6]. Joseph, R., Chong, A., Teh, M., Wee, A. & Tan, K. Thrombotic complication of umbilical arterial catheterization and its sequelae. Annals of the Academy of Medicine, Singapore 14, 576-582 (1985).
[7]. Baserga, M.C., Puri, A. & Sola, A. The use of topical nitroglycerin ointment to treat peripheral tissue ischemia secondary to arterial line complications in neonates. Journal of perinatology 22, 416 (2002).
[8]. Scheer, B., Perel, A. & Pfeiffer, U.J. Clinical review: complications and risk factors of peripheral arterial catheters used for haemodynamic monitoring in anaesthesia and intensive care medicine. Crit. Care 6, 199-204 (2002).
[9]. Chung, H.U., et al. Binodal, wireless epidermal electronic systems with in-sensor analytics for neonatal intensive care. Science 363, eaau0780 (2019).
[10]. Fanaroff, J.M. & Fanaroff, A.A. Blood pressure disorders in the neonate: hypotension and hypertension. in Seminars in Fetal and Neonatal Medicine, Vol. 11 174--181 (2006).
[11]. Wilson, R.A., Bamrah, V.S., Lindsay, J., Schwaiger, M. & Morganroth, J. Diagnostic accuracy of seismocardiography compared with electrocardiography for the anatomic and physiologic diagnosis of coronary artery disease during exercise testing. Am. J. Cardiol. 71, 536-545 (1993).
[12]. Mahoney, M.C. & Cohen, M.I. Effectiveness of developmental intervention in the neonatal intensive care unit: implications for neonatal physical therapy. Pediat. Phys. Ther. 17, 194-208 (2005).
[13]. Shinya, Y., Kawai, M., Niwa, F., Imafuku, M. & Myowa, M. Fundamental frequency variation of neonatal spontaneous crying predicts language acquisition in preterm and term infants. Front. Psychol. 8, 2195 (2017).
[14]. Boundy, E.O., et al. Kangaroo Mother Care and Neonatal Outcomes: A Meta-analysis. Pediatrics 137, e20152238 (2016).
[15]. Dehghani, K., Movahed, Z., Dehghani, H. & Nasiriani, K. A randomized controlled trial of kangaroo mother care versus conventional method on vital signs and arterial oxygen saturation rate in newborns who were hospitalized in neonatal intensive care unit. J. Clin. Neonatol. 4, 26-31 (2015).
[16]. Shwayder, T. & Akland, T. Neonatal skin barrier: structure, function, and disorders. Dermatol. Ther. 18, 87-103 (2005).
[17]. Mutashar, S., Hannan, M., Samad, S. & Hussain, A. Analysis and optimization of spiral circular inductive coupling link for bio-implanted applications on air and within human tissue. Sensors 14, 11522-11541 (2014).
[18]. Toys and Products Intended for Use By Children Under 3 Years Old. in 16 C.F.R. Part 1501 and 1500.50-53 (ed. COMMISSION, U.S.C.P.S.) (2001).
[19]. James, D.K., Dryburgh, E.H. & Chiswick, M.L. Foot length -- a new and potentially useful measurement in the neonate. Arch. Dis. Child. 54, 226-230 (1979).
[20]. Means, L.W. & Walters, R.E. Sex, handedness and asymmetry of hand and foot length. Neuropsychologia 20, 715-719 (1982).
[21]. Di Rienzo, M., et al. Wearable seismocardiography: towards a beat-by-beat assessment of cardiac mechanics in ambulant subjects. Auton. Neurosci. 178, 50-59 (2013).
[22]. Leeudomwong, T., Deesudchit, T. & Chinrungrueng, C. Motion-Resistant Pulse Oximetry Processing Based on Time-Frequency Analysis. Engineering Journal 21, 181-196 (2017).
[23]. Fu, T.-H., Liu, S.-H. & Tang, K.-T. Heart rate extraction from photoplethysmogram waveform using wavelet multi-resolution analysis. J. Med. Biol. Eng. 28, 229-232 (2008).
[24]. Daw, W., et al. Medical devices for measuring respiratory rate in children: a review. Journal of Advances in Biomedical Engineering and Technology 3, 21-27 (2016).
[25]. Bland, J.M. & Altman, D.G. Statistical methods for assessing agreement between two methods of clinical measurement. The Lancet 327, 307-310 (1986).
[26]. Cardiac Monitors, Heart Rate Meters, And Alarms. in ANSI/AAMI EC13-2002.
[27]. 21 CFR 870.2700 - Oximeter. (ed. Administration, U.S.F.D.).
[28]. Breathing Frequency Monitor. Vol. 21 CFR 870.2375 (ed. Administration, U.S.F.D.).
[29]. Lund, C.H. & Osborne, J.W. Validity and reliability of the neonatal skin condition score. Journal of Obstetric, Gynecologic, & Neonatal Nursing 33, 320-327 (2004).
[30]. Sharma, M., et al. Cuff-Less and Continuous Blood Pressure Monitoring: A Methodological Review. Technologies 5, 21 (2017).
[31]. Payne, R.A., Symeonides, C.N., Webb, D.J. & Maxwell, S.R. Pulse transit time measured from the ECG: an unreliable marker of beat-to-beat blood pressure. J. Appl. Physiol. 100, 136-141 (2006).
[32]. Foo, J.Y.A., Lim, C.S. & Wang, P. Evaluation of blood pressure changes using vascular transit time. Physiol. Meas. 27, 685-694 (2006).
[33]. Peter, L., Noury, N. & Cerny, M. A review of methods for non-invasive and continuous blood pressure monitoring: Pulse transit time method is promising? IRBM 35, 271 - 282 (2014).
[34]. Ma, Y., et al. Relation between blood pressure and pulse wave velocity for human arteries. PNAS 115, 11144-11149 (2018).
[35]. Wippermann, C.F., Schranz, D. & Huth, R.G. Evaluation of the pulse wave arrival time as a marker for blood pressure changes in critically ill infants and children. J. Clin. Monit. 11, 324-328 (1995).
[36]. Galland, B.C., Tan, E. & Taylor, B.J. Pulse transit time and blood pressure changes following auditory-evoked subcortical arousal and waking of infants. Sleep 30, 891-897 (2007).
*[37].* Kangaroo mother care: a practical guide, (World Health Organization, Dept. of Reproductive Health and Research, 2003).
[38]. Liu, G.-Z., Guo, Y.-W., Zhu, Q.-S., Huang, B.-Y. & Wang, L. Estimation of respiration rate from three-dimensional acceleration data based on body sensor network. Telemed. J. E-Health 17, 705-711 (2011).
[39]. Cattaneo, A., et al. Kangaroo mother care for low birthweight infants: a randomized controlled trial in different settings. Acta. Paediatr. 87, 976-985 (1998).
[40]. Levy, J., et al. Impact of hands-on care on infant sleep in the neonatal intensive care unit. Pediatr. Pulmonol. 52, 84-90 (2017).
[41]. Reggiannini, B., Sheinkopf, S.J., Silverman, H.F., Li, X. & Lester, B.M. A flexible analysis tool for the quantitative acoustic assessment of infant cry. J. Speech Lang. Hear. Res. 56, 1416-1428 (2013).
[42]. Liu, Y., et al. Epidermal mechano-acoustic sensing electronics for cardiovascular diagnostics and human-machine interfaces. Sci. Adv. 2, e1601185 (2016).
[43]. Huntsman, R.J., Lowry, N.J. & Sankaran, K. Nonepileptic motor phenomena in the neonate. Paediatr. Child Health 13, 680-684 (2008).
[44]. Fahn, S., Jankovic, J. & Hallett, M. Chapter 18 - Tremors. in Principles and Practice of Movement Disorders (Second Edition) (eds. Fahn, S., Jankovic, J. & Hallett, M.) 389-414 (W.B. Saunders, Edinburgh, 2011).
[45]. Pandia, K., Inan, O.T. & Kovacs, G.T.A. A frequency domain analysis of respiratory variations in the seismocardiogram signal. in 2013 35th Annual International Conference of the IEEE Engineering in Medicine and Biology Society (EMBC) 6881-6884 (2013).
[46]. Shinya, Y., Kawai, M., Niwa, F. & Myowa-Yamakoshi, M. Preterm birth is associated with an increased fundamental frequency of spontaneous crying in human infants at termequivalent age. Biol. Lett. 10(2014).
[47]. Pickering, T.G., et al. Recommendations for Blood Pressure Measurement in Humans and Experimental Animals. Circulation 111, 697-716 (2005).
[48]. Brzezinski, M., Luisetti, T. & London, M.J. Radial artery cannulation: a comprehensive review of recent anatomic and physiologic investigations. Anesth. Analg. 109, 1763--1781 (2009).
[49]. Sahoo, P.K., Thakkar, H.K. & Lee, M.-Y. A cardiac early warning system with multi channel SCG and ECG monitoring for mobile health. Sensors 17, 711 (2017).
[50]. Inan Omer, T., et al. Novel wearable seismocardiography and machine learning algorithms can assess clinical status of heart failure patients. Circ. Heart Fail. 11, e004313 (2018).
[51]. Hadush, M.Y., Berhe, A.H. & Medhanyie, A.A. Foot length, chest and head circumference measurements in detection of low birth weight neonates in Mekelle, Ethiopia: a hospital based cross sectional study. BMC Pediatr. 17, 111 (2017).
[52]. August, D.L., New, K., Ray, R.A. & Kandasamy, Y. Frequency, location and risk factors of neonatal skin injuries from mechanical forces of pressure, friction, shear and stripping: a systematic literature review. J. Neonatal Nurs. 24, 173-180 (2018).
[53]. Oranges, T., Dini, V. & Romanelli, M. Skin physiology of the neonate and infant: clinical implications. Adv. Wound Care 4, 587-595 (2015).
[54]. Barbeau, D.Y. & Weiss, M.D. Sleep disturbances in newborns. Children 4, 90 (2017).
[55]. Newman, J.D. Neural circuits underlying crying and cry responding in mammals. Behav. Brain Res. 182, 155-165 (2007).
[56]. Corwin, M.J., et al. Newborn acoustic cry characteristics of infants subsequently dying of sudden infant death syndrome. Pediatrics 96, 73-77 (1995).
[57]. Farsaie Alaie, H., Abou-Abbas, L. & Tadj, C. Cry-based infant pathology classification using GMMs. Speech Commun. 77, 28-52 (2016).
[58]. Joshi, R., *et al.* A ballistographic approach for continuous and non-obtrusive monitoring of movement in neonates. *IEEE J. Transl. Eng. Health Med.* **6**, 2700809-2700809 (2018).
[59]. Pan J & Tompkins, WJJITBE A real-time QRS detection algorithm. 32(3):230-236 (1985).
[60]. Scholkmann F, Boss J, & Wolf MJA An efficient algorithm for automatic peak detection in noisy periodic and quasi-periodic signals. 5(4):588-603 (2012).
[61]. Shensa MJJITosp, The discrete wavelet transform: wedding the a trous and Mallat algorithms. 40(10):2464-2482 (1992).
[62]. Cattaneo A, Davanzo R, Bergman N, & Charpak NJJoTP, Kangaroo mother care in lowincome countries. 44(5):279-282 (1998).
[63]. Smith LA, Dawes PJ, & Galland BC, The use of pulse transit time in pediatric sleep studies: A systematic review. Sleep Medicine Reviews (2016).
[64]. Mahoney LGS, Assessment of the transitional circulation in late preterm and term neonates using non-invasive biomarkers: a longitudinal analysis and evaluation of repeatability. (University of Brighton) (2016).

## Claims

1. An apparatus for non-invasively measuring physiological parameters of a neonatal subject, comprising:
a plurality of sensor systems attachable to the neonatal subject, wherein the plurality of sensor systems are time-synchronized and communicate with each other wirelessly and bidirectionally, wherein the plurality of sensor systems comprises:
a first epidermal electronic system (EES) attachable to a torso region of the neonatal subject, the first EES comprising an electrocardiography (ECG) sensor, an accelerometer sensor, a temperature sensor, a plurality of flexible and stretchable interconnects comprising serpentine or zig-zag interconnects, and an elastomeric encapsulation layer;
a second EES attachable to a limb region of the neonatal subject,
the second EES comprising a photoplethysmography (PPG) sensor, a temperature sensor, a plurality of flexible and stretchable interconnects comprising serpentine or zig-zag interconnects, and an elastomeric encapsulation;
wherein the first and second EES are configured to detect physiological signals that are time-synchronized with each other and to wirelessly communicate with each other; and
a microcontroller unit (MCU) adapted in wireless communication with the plurality of sensor systems, and configured to wirelessly receive the time-synchronized physiological signals from the first and second EES and to determine one or more physiological parameters of the neonatal subject based on the physiological signals, from the sensor systems, and to display the physiological parameters of the neonatal subject.

2. The apparatus of claim 1, wherein:
the first EES further comprises a foldable electronic board on which a first plurality of electronic components and the flexible and stretchable interconnects are disposed, wherein the first plurality of electronic components include one or more of the ECG sensor, the accelerometer sensor and the temperature sensor; and
the second EES further comprises a foldable electronic board on which a second plurality of electronic components and the flexible and stretchable interconnects are disposed, wherein the second plurality of electronic components include one or more of the PPG and the temperature sensor.

3. The apparatus of claim 1, wherein each EES further comprises
a power supply, the power supply being an embedded power supply or a detachable modular power supply.

4. The apparatus of claim 1,
wherein the first plurality of electronic components of the ECG sensor comprise at least two electrodes spatially apart from each other for ECG signal generation

5. The apparatus of claim 1, wherein each of the first and second EES is in wireless communication with the MCU via a near field communication (NFC) protocol, or Bluetooth protocol.

6. The apparatus of claim 1, wherein each EES is waterproof.

7. The apparatus of claim 1, wherein the physiological parameters of the neonatal
subject comprise one or more of: heart rate, respiratory rate, respiratory effort, blood oxygenation, temperature or a combination thereof.

8. The apparatus of claim 1, wherein the MCU is configured to determine a blood pressure parameter of the neonatal subject based on a pulse arrival time (PAT) between an ECG signal detected by the first EES and the PPG signal detected by the second EES, and a pulse wave velocity (PWV) determined from the pulse arrival time and a distance between the torso region and the limb region

9. The apparatus of claim 8, wherein at a blood pressure is determined according to: $P = α {PWV}^{2} + β ,$ and α and β are empirically determined constants depending on artery geometry and artery material properties.

10. A method of non-invasively measuring physiological parameters of a neonatal subject, the method comprising:
attaching a first EES epidermal electronic system (EES) comprising an electrocardiography (ECG) sensor, an accelerometer sensor, a temperature sensor, flexible and stretchable serpentine or zig-sag interconnects, and an elastomeric encapsulation layer to a torso region of the neonatal subject;
attaching a second EES comprising a photoplethysmography (PPC) sensor, a temperature sensor, flexible and stretchable serpentine or zig-zag interconnects, and an elastomeric encapsulation layer to a limb region of the neonatal subject;
detecting physiological signals at each of the first and second EES, the physiological signals being time-synchronized across the first and second EES;
wirelessly transmitting the physiological signals from the EES to a microcontroller unit; and
determining , at the microcontroller, one or more physiological parameters of the neonatal subject based on the physiological signals.

11. The method of claim 10, wherein determining the one or more physiological parameters comprises determining a blood pressure parameter
based on a pulse arrival time (PAT) between an ECG signal from the ECG sensor and a PPG signal from the PPG sensor and
a pulse wave velocity (PWV) derived from the PAT

12. The apparatus of claim 1, wherein the second plurality of electronic components of the PPG sensor comprise an optical source and an optical detector located within a footprint of the second EES.

13. The apparatus of claim 7, wherein each of the first and second EES comprises a magnetic coil configured for wireless data transmission and wireless power transmission according to the NFC protocol.

14. The apparatus of claim 1, wherein the accelerometer sensor of the first EES is configured to detect movement or posture of the neonatal subject.

15. The apparatus of claim 1, wherein the first and second EES are configured to detect chest wall displacement of the neonatal subject based on accelerometer signals from the first EES.

## Patentansprüche

1. Vorrichtung zur nicht-invasiven Messung physiologischer Parameter eines Neugeborenen, umfassend:
eine Vielzahl von Sensorsystemen, die an dem Neugeborenen angebracht werden können, wobei die Vielzahl von Sensorsystemen zeitlich synchronisiert ist und drahtlos sowie bidirektional miteinander kommuniziert, wobei die Vielzahl von Sensorsystemen umfasst:
ein erstes epidermales elektronisches System (EES), das an einem Rumpfbereich des Neugeborenen angebracht werden kann, wobei das erste EES einen Elektrokardiographie-Sensor (EKG-Sensor), einen Beschleunigungssensor, einen Temperatursensor, eine Vielzahl flexibler und dehnbarer Verbindungen, die Serpentinen- oder Zickzack-Verbindungen umfassen, und eine elastomere Verkapselungsschicht umfasst;
ein zweites EES, das an einem Gliedmaßenbereich des Neugeborenen angebracht werden kann, wobei das zweite EES einen Photoplethysmographie-Sensor (PPG-Sensor), einen Temperatursensor, eine Vielzahl flexibler und dehnbarer Verbindungen, die Serpentinen- oder Zickzack-Verbindungen umfassen, und eine elastomere Verkapselung umfasst;
wobei das erste und das zweite EES so konfiguriert sind, dass sie physiologische Signale erfassen, die zeitlich miteinander synchronisiert sind, und drahtlos miteinander zu kommunizieren; und
eine Mikrocontroller-Einheit (MCU), die für eine drahtlose Kommunikation mit der Vielzahl von Sensorsystemen ausgelegt ist und so konfiguriert ist, dass sie die zeitlich synchronisierten physiologischen Signale von der ersten und der zweiten EES drahtlos empfängt, einen oder mehrere physiologische Parameter des Neugeborenen auf der Grundlage der physiologischen Signale aus den Sensorsystemen ermittelt und die physiologischen Parameter des Neugeborenen anzeigt.

2. Vorrichtung nach Anspruch 1, wobei:
das erste EES ferner eine faltbare Elektronikplatine umfasst, auf der eine erste Vielzahl von elektronischen Komponenten und die flexiblen und dehnbaren Verbindungen angeordnet sind, wobei die erste Vielzahl von elektronischen Komponenten einen oder mehrere der folgenden Sensoren beinhaltet: den EKG-Sensor, den Beschleunigungssensor und den Temperatursensor; und
das zweite EES ferner eine faltbare Elektronikplatine umfasst, auf der eine zweite Vielzahl von elektronischen Komponenten und die flexiblen und dehnbaren Verbindungen angeordnet sind, wobei die zweite Vielzahl von elektronischen Komponenten einen oder mehrere der PPG- und Temperatursensoren beinhaltet.

3. Vorrichtung nach Anspruch 1, wobei jedes EES ferner umfasst:
eine Stromversorgung, wobei die Stromversorgung eine integrierte Stromversorgung oder eine abnehmbare modulare Stromversorgung ist.

4. Vorrichtung nach Anspruch 1,
wobei die erste Vielzahl elektronischer Komponenten des EKG-Sensors mindestens zwei räumlich voneinander getrennte Elektroden zur Erzeugung eines EKG-Signals umfasst.

5. Vorrichtung nach Anspruch 1, wobei sowohl das erste als auch das zweite EES über ein Nahfeldkommunikationsprotokoll (NFC-Protokoll) oder ein Bluetooth-Protokoll in drahtloser Kommunikation mit der MCU steht.

6. Vorrichtung nach Anspruch 1, wobei jedes EES wasserdicht ist.

7. Vorrichtung nach Anspruch 1, wobei die physiologischen Parameter des Neugeborenen einen oder mehrere der folgenden Parameter umfassen: Herzfrequenz, Atemfrequenz, Atemarbeit, Blutsauerstoffsättigung, Temperatur oder eine Kombination davon.

8. Vorrichtung nach Anspruch 1, wobei die MCU so konfiguriert ist, dass sie einen Blutdruckparameter des Neugeborenen auf der Grundlage einer Pulsankunftszeit (PAT) zwischen einem von dem ersten EES erfassten EKG-Signal und dem von dem zweiten EES erfassten PPG-Signal sowie eine Pulswellengeschwindigkeit (PWV) bestimmt, die aus der Pulsankunftszeit und einem Abstand zwischen dem Rumpfbereich und dem Gliedmaßenbereich ermittelt wird.

9. Vorrichtung nach Anspruch 8, wobei ein Blutdruck gemäß folgender Gleichung bestimmt wird: $P = α {PWV}^{2} + β ,$ wobei α und β empirisch ermittelte Konstanten sind, die von der Arteriengeometrie und Materialeigenschaften der Arterien abhängen.

10. Verfahren zur nicht-invasiven Messung physiologischer Parameter eines Neugeborenen, wobei das Verfahren umfasst:
Anbringen eines ersten epidermalen elektronischen Systems (EES), das einen Elektrokardiographie-Sensor (EKG-Sensor), einen Beschleunigungssensor, einen Temperatursensor, flexible und dehnbare serpentinenförmige oder zickzackförmige Verbindungen sowie eine elastomere Verkapselungsschicht umfasst, an einem Rumpfbereich des Neugeborenen;
Anbringen eines zweiten EES, das einen Photoplethysmographie-Sensor (PPC-Sensor), einen Temperatursensor, flexible und dehnbare, aserpentinenförmige oder zickzackförmige Verbindungen sowie eine elastomere Verkapselungsschicht umfasst, an einem Gliedmaßenbereich des Neugeborenen;
Erfassen physiologischer Signale sowohl an dem ersten als auch dem zweiten EES, wobei die physiologischen Signale über das erste und zweite EES zeitlich synchronisiert sind;
drahtloses Übertragen der physiologischen Signale von den EES an eine Mikrocontroller-Einheit; und
Bestimmen eines oder mehrerer physiologischer Parameter des Neugeborenen an dem Mikrocontroller anhand der physiologischen Signale.

11. Verfahren nach Anspruch 10, wobei das Bestimmen des einen oder der mehreren physiologischen Parameter das Bestimmen eines Blutdruckparameters umfasst,
basierend auf einer Pulsankunftszeit (PAT) zwischen einem EKG-Signal vom EKG-Sensor und einem PPG-Signal von dem PPG-Sensor und
einer aus der PAT abgeleiteten Pulswellengeschwindigkeit (PWV)

12. Vorrichtung nach Anspruch 1, wobei die zweite Vielzahl elektronischer Komponenten des PPG-Sensors eine optische Quelle und einen optischen Detektor umfasst, die innerhalb einer Ausdehnung des zweiten EES angeordnet sind.

13. Vorrichtung nach Anspruch 7, wobei sowohl das erste als auch das zweite EES eine Magnetspule umfasst, die für eine drahtlose Datenübertragung und drahtlose Energieübertragung gemäß dem NFC-Protokoll ausgelegt ist.

14. Vorrichtung nach Anspruch 1, wobei der Beschleunigungssensor des ersten EES so ausgelegt ist, dass er Bewegungen oder Körperhaltung des Neugeborenen erfasst.

15. Vorrichtung nach Anspruch 1, wobei das erste und das zweite EES so ausgelegt sind, dass sie eine Verschiebung der Brustwand des Neugeborenen auf der Grundlage von Beschleunigungssignalen von dem ersten EES erfassen.

## Revendications

1. Dispositif destiné à mesurer de manière non invasive des paramètres physiologiques d'un sujet néonatal, comprenant :
une pluralité de systèmes de capteurs susceptibles d'être fixés au sujet néonatal, dans lequel la pluralité de systèmes de capteurs est synchronisée dans le temps et communique entre eux sans fil et de manière bidirectionnelle, et dans lequel la pluralité de systèmes de capteurs comprend :
un premier système électronique épidermique (EES) susceptibles d'être fixés à une région du torse du sujet néonatal, le premier EES comprenant un capteur d'électrocardiographie (ECG), un capteur accélérométrique, un capteur de température, une pluralité d'interconnexions flexibles et extensibles comprenant des interconnexions en serpentine ou en zigzag, et une couche d'encapsulation en élastomère ;
un deuxième EES susceptibles d'être fixés à une région d'un membre du sujet néonatal, le deuxième EES comprenant un capteur de photopléthysmographie (PPG), un capteur de température, une pluralité d'interconnexions flexibles et extensibles comprenant des interconnexions en serpentine ou en zigzag, et une encapsulation en élastomère ;
dans lequel les premier et deuxième EES sont configurés pour détecter des signaux physiologiques synchronisés dans le temps les uns par rapport aux autres et pour communiquer sans fil entre eux ; et
une unité de microcontrôleur (MCU) adaptée pour communiquer sans fil avec la pluralité de systèmes de capteurs, et configurée pour recevoir sans fil les signaux physiologiques synchronisés dans le temps provenant des premier et deuxième EES, et pour déterminer un ou plusieurs paramètres physiologiques du sujet néonatal sur la base des signaux physiologiques provenant des systèmes de capteurs, et pour afficher les paramètres physiologiques du sujet néonatal.

2. Dispositif selon la revendication 1, dans lequel :
le premier EES comprend en outre une carte électronique pliable sur laquelle sont disposés une première pluralité de composants électroniques et les interconnexions flexibles et extensibles, dans lequel la première pluralité de composants électroniques inclut un ou plusieurs des éléments suivants : le capteur ECG, le capteur accélérométrique et le capteur de température ; et
le deuxième EES comprend en outre une carte électronique pliable sur laquelle sont disposés une deuxième pluralité de composants électroniques et les interconnexions flexibles et extensibles, dans lequel la deuxième pluralité de composants électroniques inclut un ou plusieurs des éléments suivants : le capteur PPG et le capteur de température.

3. Dispositif selon la revendication 1, dans lequel chaque EES comprend en outre
une alimentation électrique, l'alimentation électrique étant une alimentation intégrée ou une alimentation modulaire amovible.

4. Dispositif selon la revendication 1,
dans lequel la première pluralité de composants électroniques du capteur ECG comprend au moins deux électrodes espacées l'une de l'autre pour la génération d'un signal ECG

5. Dispositif selon la revendication 1, dans lequel chacun des premier et deuxième EES est en communication sans fil avec l'unité de microcontrôleur (MCU) via un protocole de communication en champ proche (NFC) ou un protocole Bluetooth.

6. Dispositif selon la revendication 1, dans lequel chaque EES est étanche.

7. Dispositif selon la revendication 1, dans lequel les paramètres physiologiques du sujet néonatal comprennent un ou plusieurs des éléments suivants : la fréquence cardiaque, la fréquence respiratoire, l'effort respiratoire, l'oxygénation sanguine, la température ou une combinaison de ceux-ci.

8. Dispositif selon la revendication 1, dans lequel l'unité de microcontrôleur (MCU) est configurée pour déterminer un paramètre de pression artérielle du sujet néonatal sur la base d'un temps d'arrivée du pouls (PAT) entre un signal ECG détecté par le premier EES et le signal PPG détecté par le deuxième EES, et d'une vitesse d'onde de pouls (PWV) déterminée à partir du temps d'arrivée du pouls et d'une distance entre la région du torse et la région du membre.

9. Dispositif selon la revendication 8, dans lequel la pression artérielle est déterminée selon : $P = α {PWV}^{2} + β ,$ où α et β sont des constantes déterminées empiriquement en fonction de la géométrie de l'artère et des propriétés du matériau de l'artère.

10. Procédé de mesure non invasive de paramètres physiologiques d'un sujet néonatal, le procédé comprenant :
la fixation d'un premier système électronique épidermique (EES) comprenant un capteur d'électrocardiographie (ECG), un capteur accélérométrique, un capteur de température, des interconnexions flexibles et extensibles en forme de serpentine ou en zigzag, et une couche d'encapsulation en élastomère sur une région du torse du sujet néonatal ;
la fixation d'un deuxième EES comprenant un capteur de photopléthysmographie (PPG), un capteur de température, des interconnexions flexibles et extensibles en forme de serpentine ou en zigzag, et une couche d'encapsulation en élastomère sur une région d'un membre du sujet néonatal ;
la détection de signaux physiologiques au niveau de chacun des premier et deuxième EES, les signaux physiologiques étant synchronisés dans le temps entre les premier et deuxième EES ;
la transmission sans fil des signaux physiologiques depuis les EES vers une unité de microcontrôleur ; et
la détermination au niveau de l'unité de microcontrôleur d'un ou de plusieurs paramètres physiologiques du sujet néonatal sur la base des signaux physiologiques.

11. Procédé selon la revendication 10, dans lequel la détermination du ou des paramètres physiologiques comprend la détermination d'un paramètre de pression artérielle sur la base d'un temps d'arrivée du pouls (PAT) entre un signal ECG provenant du capteur ECG et un signal PPG provenant du capteur PPG, et
d'une vitesse d'onde de pouls (PWV) dérivée du PAT

12. Dispositif selon la revendication 1, dans lequel la deuxième pluralité de composants électroniques du capteur PPG comprend une source optique et un détecteur optique situés à l'intérieur de l'empreinte du deuxième EES.

13. Dispositif selon la revendication 7, dans lequel chacun des premier et deuxième EES comprend une bobine magnétique configurée pour la transmission sans fil de données et la transmission sans fil d'énergie selon le protocole NFC.

14. Dispositif selon la revendication 1, dans lequel le capteur accélérométrique du premier EES est configuré pour détecter le mouvement ou la posture du nouveau-né.

15. Dispositif selon la revendication 1, dans lequel les premier et deuxième EES sont configurés pour détecter le déplacement de la paroi thoracique du nouveau-né sur la base des signaux de l'accéléromètre provenant du premier EES.
